# EUROPEAN PATENT APPLICATION

(11) **EP 4 159 735 A1**
(43) Date of publication of application: **05.04.2023**
(21) Application number: 21812993.0
(22) Date of filing: 21.05.2021
(51) Int. Cl.: C07D 471/18, A61K 31/505, A61P 35/00

(54) **MACROCYCLIC JAK INHIBITOR AND USE THEREOF**

(30) Priority: 29.05.2020 CN 202010477110
(71) Applicant: Biopolar Hongye (Guangdong) Pharmaceutical Co. Ltd, Zhongshan, Guangdong 528437 (CN); Biopolar Hongye (Nantong) Pharmaceutical Co., Ltd, Nantong, Jiangsu 226133 (CN)
(72) Inventor: LV, Zhijian, Nantong, Jiangsu 226133 (CN); LI, Jia, Nantong, Jiangsu 226133 (CN); SU, Mingbo, Nantong, Jiangsu 226133 (CN); GAO, Anhui, Nantong, Jiangsu 226133 (CN)
(74) Representative: Lavoix
(86) International application number: PCT/CN2021/095329
(87) International publication number: WO 2021/238817

(57) **Abstract**

The present invention relates to a macrocyclic JAK inhibitor and the use thereof. The present invention specifically relates to a compound as shown in formula I, or a stereoisomer or optical isomer thereof, a pharmaceutically acceptable salt thereof, and a prodrug or solvate thereof, and also relates to a pharmaceutical composition of the compound and the medical use thereof as a JAK inhibitor, and in the preparation of medicaments for preventing and/or treating diseases related to JAK, especially JAK1.

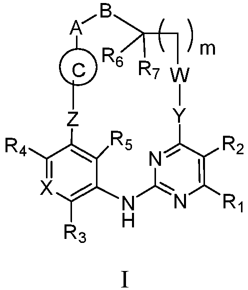

## Description

### TECHNICAL FIELD

The invention belongs to the field of medicinal chemistry, and specifically relates to a macrocyclic JAK inhibitor and use thereof.

### BACKGROUND OF THE INVENTION

Protein kinases (PK) are a group of enzymes that regulate a variety of important biological processes that constitute one of the largest families of enzymes in human. In particular, the biological processes include cellular kinases that catalyze the phosphorylation of proteins, lipids, sugars, nucleosides and other cellular metabolites and play a key role in all aspects of eukaryotic cell physiology. It has been shown that abnormal kinase activity is involved in many human diseases, including cancer, autoimmune diseases and inflammatory diseases.

Janus kinase (JAK) is a cytoplasmic tyrosine kinase that transduces cytokine signals from membrane receptors to STAT transcription factors, and plays an important role in cytokine signaling. The JAK family includes four members JAK1, JAK2, JAK3 and tyrosine kinase 2(TYK2). JAK usually associates with cytokine receptors in pairs as homodimers or heterodimers. Cytokines bind to their receptors, causing dimerization of receptor molecules. Receptor-coupled JAKs approach each other and are activated by phosphorylation of interacting tyrosine residues. JAK family transmits cytokine-mediated signals to cells through JAK-STAT (signal transduction and transcription activation factor) pathway.

Signal Transducer and Activator of Transcription (STAT) are a group of cytoplasmic proteins that can bind to target gene regulatory region DNA. As the downstream substrate of JAKs, STATs can be activated by tyrosine phosphorylation under the stimulation of external signals, and then transferred to nucleus to regulate the transcription of genes. When cytokine bind to its receptor, JAK family members autophosphorylate and/or transphosphorylate each other, followed by STATs phosphorylation, and then migrate into the nucleus to regulate transcription.

Many abnormal immune responses, such as allergies, asthma, (allogeneic) transplant rejection, rheumatoid arthritis, amyotrophic lateral sclerosis and multiple sclerosis and other autoimmune diseases, myelodysplasia, hematologic malignancies such as leukemia and lymphoma, have their regulation associated with the JAK/STAT signaling pathway.

Studies have shown that blocking signal transduction at the level of JAK kinase provides prospects for the development of therapeutic methods for inflammatory diseases, autoimmune diseases, myeloproliferative diseases and cancer. The inhibition of JAK kinase also contributes to the treatment of skin immune diseases such as psoriasis and skin sensitization. Pfizer's Toficitinib has been marketed for the treatment of rheumatoid arthritis; and Incyte's Ruxolitinib for the treatment of myelofibrosis and acute graft-versus-host disease.

However, some of the currently available JAK kinase inhibitors also have some significant toxic side effects. For example, some JAK inhibitors are prone to the following side effects: infections, including pneumonia, viral infections (such as herpes zoster infection), bacterial infections, actinomycotic infections (mycobacterial infections), fungal infections, decreased immunity (such as NK cell reduction), and anemia. In the United States, there are even black box warnings for some serious side effects, such as acute tuberculosis, invasive fungal infections, bacterial infections, and some lymphoma or other tumors. Studies have shown that the existing JAK inhibitors often have inhibitory activity on JAK1 and JAK3, and most of these side effects are related to the inhibition of JAK3 activity.

However, studies have shown that JAKs family kinases are responsible for regulating many signaling pathways. Since JAK1 and JAK3 are components of common γ-chain cytokine receptor complexes, it is very difficult to develop inhibitors with high selectivity to JAK1.

JAK1 plays a key role in biological response regulation, and JAK1 is widely expressed and associated with several major cytokine receptor families. It is involved in signaling through members of the IL-2 receptor gamma subunit family (IL-2, IL-4, IL-7R, IL-9R, IL-15R and IL-21R), IL-4 receptor family (IL-4R, IL-13R), gp130 receptor family and class II cytokine receptors (including IL-10 receptor family and both type I and type II IFN receptor families).

In summary, there is an urgent need in the art to develop inhibitors of Janus kinase or related kinases, especially inhibitors with high selectivity to JAK1.

### SUMMARY OF THE INVENTION

The invention provides an inhibitor of JAK or related kinases, especially an inhibitor with high selectivity to JAK1.

In the first aspect of the present invention, it provides a compound of formula I or a stereoisomer or optical isomer, a pharmaceutically acceptable salt, a prodrug or a solvate thereof, 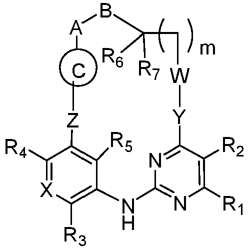 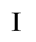 wherein,
A is independently selected from the group consisting of C(=O), -C(=O)O-, C(=O)NH, N-R_{b}, O, S, SO, and SO₂;
B is independently selected from the group consisting of bond, C(=O), N-R_{b}, C(R_{c})₂, C(= O)O-, O, S, SO, and SO₂;
ring C is independently selected from the substituted or unsubstituted group consisting of 3-10 membered heterocycloalkyl, C3-C10 cycloalkyl, 5-12 membered heteroaryl, and C6-C12 aryl;
X is independently selected from the group consisting of N and CR_{d};
Y is independently selected from the group consisting of C(=O), N-R_{b}, C(R_{c})₂, C(=O)O-, O, S, SO, and SO₂;
Z is independently selected from the group consisting of bond, N-R_{b}, O, S, and C(R_{c})₂;
W is independently selected from the group consisting of C(R_{c})₂, 3-10 membered heterocycloalkyl, C3-C10 cycloalkyl, 5-12 membered heteroaryl, C6-C12 aryl, and L₁-(CH₂)ₙ-L₂; wherein L₁ is independently selected from the group consisting of absent, 3-10 membered heterocycloalkyl, C3-C10 cycloalkyl, 5-12 membered heteroaryl, and C6-C12 aryl, L₂ is independently selected from the group consisting of N-R_{b}, C(=O)O-, O, S, SO, and SO₂;
R_{b} is independently selected from the group consisting of H, and C1-C6 alkyl;
R_{c} is each independently selected from the group consisting of H, halogen, amino, nitro, hydroxyl, cyano, carboxyl, sulfonyl, sulfinyl, amido, sulfonamido, ester group, C1-C6 alkyl, C1-C6 alkoxy, 3-10 membered heterocycloalkyl, C3-C10 cycloalkyl, 5-12 membered heteroaryl, and C6-C12 aryl;
R₁, R₂, R₃, R₄, R₅, R₆, R₇ and R_{d} are each independently selected from the group consisting of H, D, halogen, amino, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
or, R₁ and R₂ together with the atoms to which they are attached form the substituted or unsubstituted group consisting of 5-6 membered aryl or heteroaryl, 3-10 membered heterocyclyl, and C3-C10 cycloalkyl;
the H atom in -(CH₂)ₘ- and -(CH₂)ₙ- can be optionally substituted by one or more Rₐ;
the "substituted" refers to being substituted by one or more (such as 2, 3, 4, 5) groups selected from the group consisting of D, halogen, amino, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more (such as 2, 3, 4, 5) Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
m is 1, 2, 3, 4, 5 or 6;
n is 0, 1, 2, or 3.

In another preferred embodiment, the compound or stereoisomer or optical isomer, pharmaceutically acceptable salt, prodrug or solvate has a structure shown in formula II:

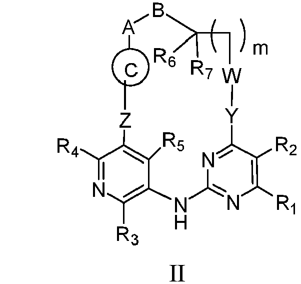

A, B, C, W, Y, Z, R₁, R₂, R₃, R₄, R₅, R₆, R₇ and m are defined as above.

In another preferred embodiment, Z is bond.

In another preferred embodiment, Y is NH.

In another preferred embodiment, the compound or stereoisomer or optical isomer, pharmaceutically acceptable salt, prodrug or solvate thereof has a structure shown in formula III: 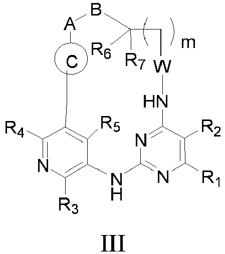 wherein, A, B, C, W, R₁, R₂, R₃, R₄, R₅, R₆, R₇, and m are defined as above.

In another preferred embodiment, in the compound or the stereoisomer or optical isomer, pharmaceutically acceptable salt, prodrug or solvate thereof, W is independently selected from the group consisting of C(R_{c})₂, 6 membered heterocycloalkyl, C6 cycloalkyl, 6 membered aryl or heteroaryl, and Lᵢ-(CH₂)ₙ-L₂; wherein L₁ is independently selected from the group consisting of absent, 6 membered heterocycloalkyl, C6 cycloalkyl, and 6 membered aryl or heteroaryl, L₂ is independently selected from the group consisting of N-R_{b}, C(=O)O-, O, S, SO, and SO₂;
the above heterocycloalkyl, cycloalkyl, heteroaryl, and aryl may be further optionally substituted by one or more (such as 2, 3, 4, 5) Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
R_{b}, R_{c}, and n are defined as above.

In another preferred embodiment, L₁ is connected to Y.

In another preferred embodiment, R₃ is halogen.

In another preferred embodiment, A is C(=O).

In another preferred embodiment, A is N-R_{b}; wherein R_{b} is independently selected from the group consisting of H, and C1-C6 alkyl.

In another preferred embodiment, A is -C(=O)NH-, and B is bond.

In another preferred embodiment, ring C is independently selected from the substituted or unsubstituted group consisting of 6-membered heterocycloalkyl, C6 cycloalkyl, 6 membered heteroaryl, and C6 aryl; wherein, the "substituted" refers to being substituted by one or more groups selected from the group consisting of D, halogen, amino, nitro, hydroxyl, sulfydryl, cyano, carboxyl, sulfydryl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, the compound or the stereoisomer or optical isomer, pharmaceutically acceptable salt, prodrug or solvate thereof, wherein the ring C is selected from the group consisting of 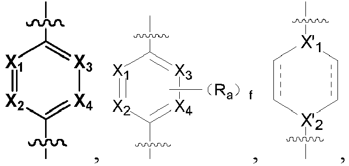
wherein, -̅-̅-̅-̅-̅ represents single bond or double bond;
X₁, X₂, X₃, X₄, X'₁ and X'₂ are each independently selected from the group consisting of N, and C-R_{d}; and 0, 1, 2 or 3 of X₁, X₂, X₃, and X₄ are N;
R_{d} is defined as above.

In another preferred embodiment, both X'₁ and X'₂ are N.

In another preferred embodiment, the ring C is selected from 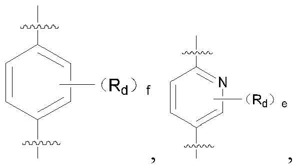 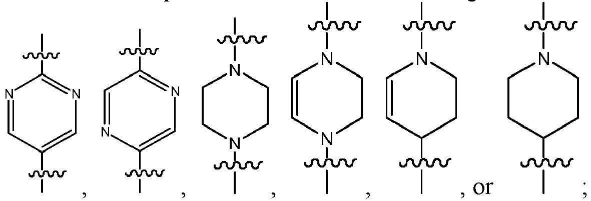
R_{d} is each independently selected from the group consisting of H, D, halogen, amino, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
e is 0, 1, 2 or 3;
f is 0, 1, 2, 3 or 4.

In another preferred embodiment, the compound has a structure shown in formula IV-1 and IV-2: 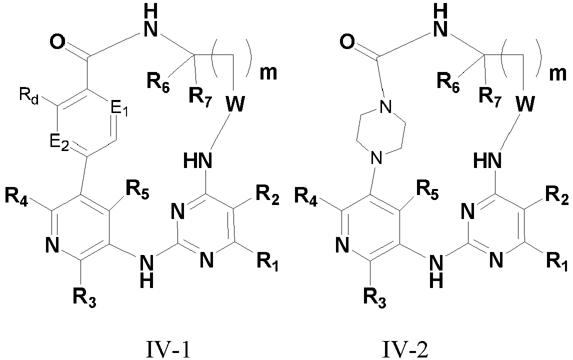 wherein,
E₁ and E₂ are each independently N or CR_{d};
R₁, R₂, R₃, R₄, R₅, R₆, R₇, R_{d}, and m are defined as above.

In another preferred embodiment, E₁ is N.

In another preferred embodiment, E₂ is N.

In another preferred embodiment, m is 1, 2, 3 or 4.

In another preferred embodiment, n is 1.

In another preferred embodiment, the compound of formula I has one or more of the following characteristics:
A is C(= O), C(= O)NH or N-R_{b}; wherein, R_{b} is independently selected from the group consisting of H and C1-C6 alkyl;
B is bond or N-R_{b};
ring C is independently selected from the substituted or unsubstituted group consisting of 6 membered heterocycloalkyl, C6 cycloalkyl, 6 membered heteroaryl, and C6 aryl; preferably,
ring C is 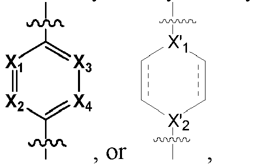
wherein, -̅-̅-̅-̅-̅ represents single bond or double bond;
X₁, X₂, X₃, X₄, X'₁ and X'₂ are each independently selected from the group consisting of N, and C-R_{d}; and 0, 1, 2 or 3 of X₁, X₂, X₃, and X₄ are N;
wherein, the "substituted" refers to being substituted by one or more groups selected from the group consisting of D, halogen, amino, nitro, hydroxyl, sulfydryl, cyano, carboxyl, sulfydryl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
W is independently selected from the group consisting of C(R_{c})₂, 6 membered heterocycloalkyl, C6 cycloalkyl, 6 membered aryl or heteroaryl, and Lᵢ-(CH₂)ₙ-L₂; wherein L₁ is independently selected from the group consisting of absent, 6 membered heterocycloalkyl, C6 cycloalkyl, and 6 membered aryl or heteroaryl, L₂ is independently selected from the group consisting of N-R_{b}, C(=O)O-, O, S, SO, and SO₂;
XisN;
Y is N-R_{b};
Z is bond;
R₁, R₂, R₃, R₄, R₅, R₆, R₇ and R_{d} are each independently selected from the group consisting of H, D, halogen, amino, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
R_{c} is each independently selected from the group consisting of H, halogen, amino, nitro, hydroxyl, cyano, carboxyl, sulfonyl, sulfinyl, amido, sulfonamido, ester group, C1-C6 alkyl, C1-C6 alkoxy, 3-10 membered heterocycloalkyl, C3-C10 cycloalkyl, 5-12 membered heteroaryl, and C6-C12 aryl;
the above alkyl, alkoxy, alkenyl, alkynyl, heterocycloalkyl, cycloalkyl, heteroaryl, and aryl may be further optionally substituted by one or more (such as 2, 3, 4, 5) Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, -A-B- is -C(=O)NH-.

In another preferred embodiment, Y is NH.

In another preferred embodiment, W is independently selected from the group consisting of CH₂, and substituted phenyl; wherein, the "substituted" refers to being substituted by 1-3 groups selected from the group consisting of halogen, amino, hydroxyl, sulfydryl, cyano, carboxyl, sulfonyl, sulfinyl, amido, sulfonamido, ester group, formyl, formamido, C1-C6 alkyl, and C1-C6 alkoxy.

In another preferred embodiment, R₆ and R₇ are each independently selected from the group consisting of H, D, halogen, and C1-C6 alkyl.

In another preferred embodiment, R_{d} is selected from the group consisting of H, D, halogen, C1-C6 alkyl, C1-C6 alkoxy, amino, nitro, RₘNRₚ, RₘSO₂-, -COORₚ, -CONR_{y}R_{z}, and -NR_{y}CORₘ; Rₘ is independently selected from C1-C6 alkyl, 3-10 membered heterocycloalkyl, C3-C10 cycloalkyl, 5-12 membered heteroaryl, or C6-C12 aryl; Rₚ is independently selected from H, C1-C6 alkyl, 3-10 membered heterocycloalkyl, C3-C10 cycloalkyl, 5-12 membered heteroaryl, or C6-C12 aryl; R_{y} and R_{z} are each independently selected from H, C1-C6 alkyl, C1-C6 alkoxy, 3-10 membered heterocycloalkyl, C3-C10 cycloalkyl, 5-12 membered heteroaryl, or C6-C12 aryl; wherein, the alkyl, alkoxy, alkenyl, alkynyl, heterocycloalkyl, cycloalkyl, heteroaryl, and aryl may be further optionally substituted by one or more Rₐ, wherein each Rₐ is independently selected from the group consisting of halogen, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

In another preferred embodiment, A, B, C, W, X, Y, Z, R₁, R₂, R₃, R₄, R₅, R₆, R₇, and m are the specific groups corresponding to each specific compound in the example.

In another preferred embodiment, the compound or the stereoisomer or optical isomer, pharmaceutically acceptable salt, prodrug or solvate thereof, wherein the compound is selected from the group consisting of

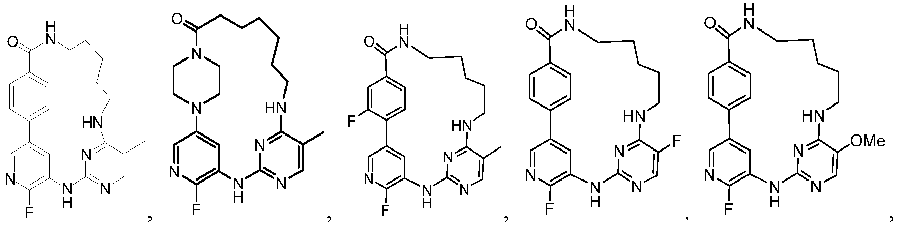

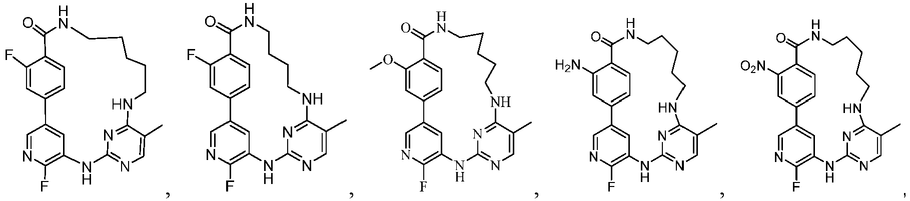

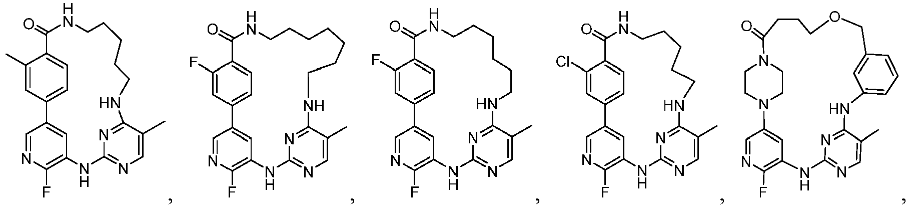

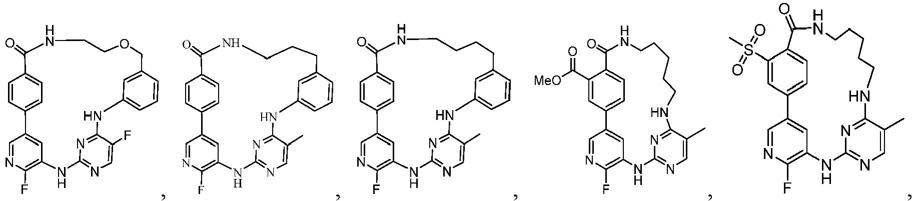

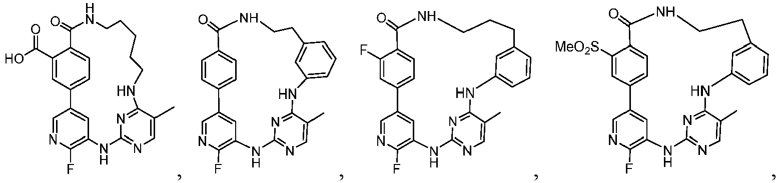

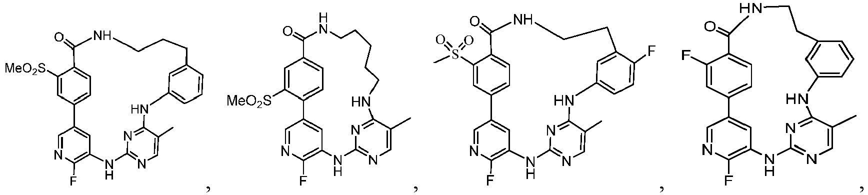

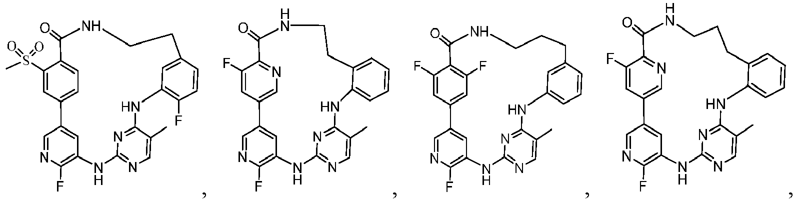

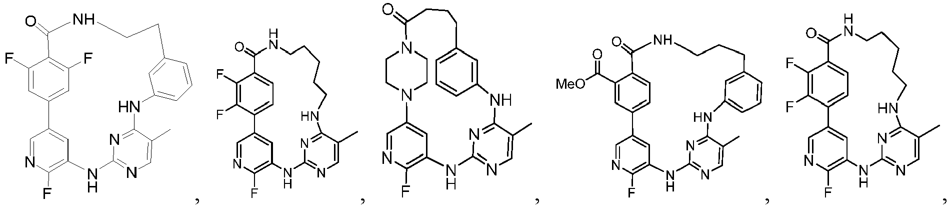

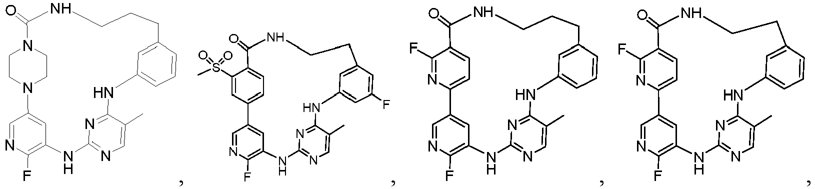

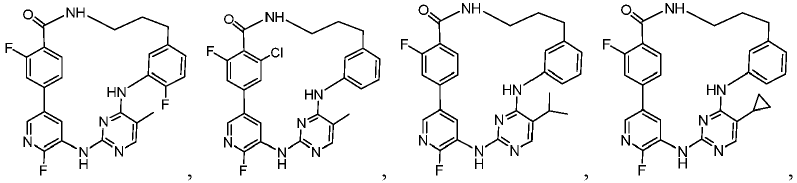

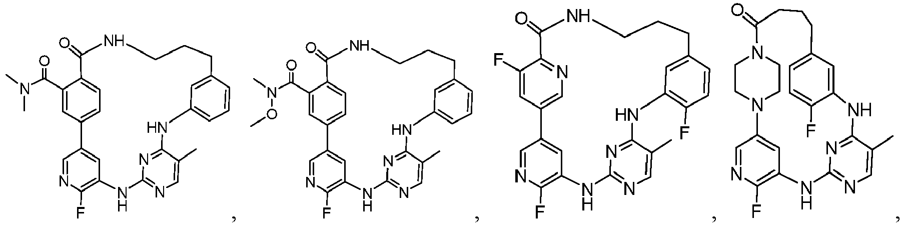

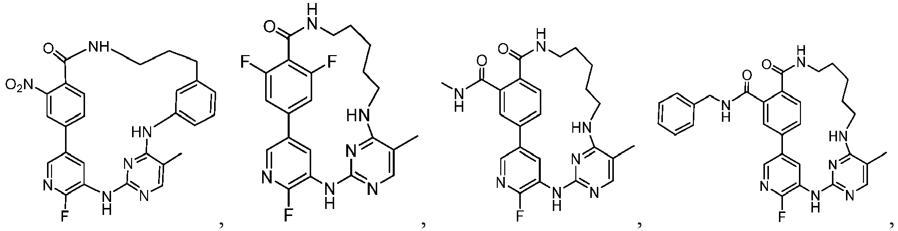

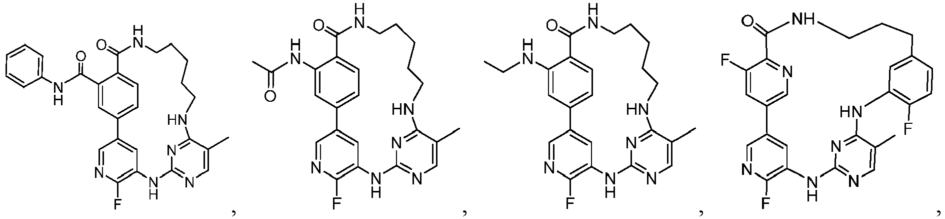

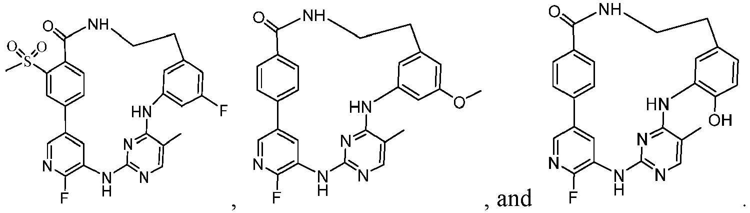

In another preferred embodiment, the compound or the stereoisomer or optical isomer, pharmaceutically acceptable salt, prodrug or solvate thereof, wherein the compound is selected from the compounds shown in the examples.

In the second aspect of the invention, it provides a pharmaceutical composition comprising the compound or the stereoisomer or optical isomer, pharmaceutically acceptable salt, prodrug or solvate thereof of the first aspect, and a pharmaceutically acceptable carrier.

In another preferred embodiment, the pharmaceutical composition further comprises

In another preferred embodiment, the pharmaceutical composition further comprises a drug selected from the group consisting of
PD-1 inhibitor (e. g., nivolumab, pimumab, pidilizumab, cemiplimab, JS-001, SHR-120, BGB-A317, IBI-308, GLS-010, GB-226, STW204, HX008, HLX10, BAT1306, AK105, LZM 009 or the biological analogue thereof, etc.), PD-L1 inhibitor (e. g, dulvalumab, atezumab, avelumab, CS1001, KN035, HLX20, SHR-1316, BGB-A333, JS003, CS1003, KL -A167, F 520, GR1405, MSB2311 or the biological analogue thereof, etc.), CD20 antibody (e. g, rituximab, obinutuzumab, ofatumumab, veltuzumab, tositumomab, 1311-tositumomab, ibritumomab tiuxetan, 90Y-ibritumomab tiuxetan, 90In-ibritumomab tiuxetan, ibritumomab tiuxetan, etc.), CD47 antibody (e. g, Hu5F9-G4, CC-90002, TTI-621, TTI-622, OSE-172, SRF-231, ALX-148, NI-1701, SHR-1603, IBI188, IMM01), ALK inhibitor (e. g, Ceritinib, Alectinib, Brigatinib, Lorlatinib, Ocatinib), PI3K inhibitors (e. g, Idelalisib, Duvelisib, Dactolisib, Taselisib, Bimiralisib, Omipalisib, Buparlisib, etc.), BTK inhibitor (e. g, ibrutinib, Tirabrutinib, Acalabrutinib, Zanubrutinib, Vecabrutinib, etc.), EGFR inhibitor (e. g, Afatinib, Gefitinib, Erlotinib , Lapatinib, Dacomitinib, Icotinib, Canertinib, Sapitinib, Naquotinib, Pyrotinib, Rociletinib, Osimertinib, etc.), VEGFR inhibitor (e. g, Sorafenib, Pazopanib, Regorafenib, Sitravatinib, Ningetinib, Cabozantinib, Sunitinib, Donafenib, etc.), HDAC inhibitor (e. g, Givinostat, Tucidinostat, Vorinostat, Fimepinostat, Droxinostat, Entinostat, Dacinostat, Quisinostat, Tacedinaline, etc.), CDK inhibitor (e. g, Palbociclib, Ribociclib, Abemaciclib, Milciclib, Trilaciclib, Lerociclib, etc.), MEK inhibitor (e. g, Simetinib (AZD6244), Trametinib (GSK1120212), PD0325901, U0126, Pimasertib (AS-703026), PD184352 (CI-1040), etc.), mTOR inhibitor (e. g, Vistusertib, etc.), SHP2 inhibitor (e. g, RMC-4630, JAB-3068, TNO155, etc.), and a combination thereof.

In another preferred embodiment, there is provided a method for preparing a pharmaceutical composition, comprising the step of mixing a pharmaceutically acceptable carrier with the compound or the stereoisomer or optical isomer, pharmaceutically acceptable salt, prodrug or solvate of the first aspect of the present invention, thereby forming the pharmaceutical composition.

In another preferred embodiment, the compound of the present invention can be prepared into powder, tablet, granule, capsule, solution, emulsion, suspension and the like.

In another preferred embodiment, the pharmaceutical composition is used to treat or prevent diseases related to the activity or expression of JAK kinase.

In another preferred embodiment, the disease is selected from the group consisting of cancer, myeloproliferative disease, inflammation, immune disease, organ transplantation, viral disease, cardiovascular disease or metabolic disease, human or animal autoimmune disease, rheumatoid arthritis, skin disease, multiple sclerosis, rheumatoid arthritis, psoriatic arthritis, inflammatory bowel disease, myasthenia gravis, and psoriasis.

In the third aspect of the present invention, it provides a use of the compound, or the stereoisomer or optical isomer, pharmaceutically acceptable salt, prodrug or solvate thereof of the first aspect for preparing a medicament or a pharmaceutical composition for treating or preventing a disease related to the activity or expression of JAK kinase.

In another preferred embodiment, the disease is selected from the group consisting of cancer, myeloproliferative disease, inflammation, immune disease, organ transplantation, viral disease, cardiovascular disease or metabolic disease, human or animal autoimmune disease, rheumatoid arthritis, skin disease, multiple sclerosis, rheumatoid arthritis, psoriatic arthritis, inflammatory bowel disease, myasthenia gravis, and psoriasis.

In another preferred embodiment, the cancer is selected from the group consisting of prostate cancer, kidney cancer, liver cancer, breast cancer, lung cancer, thyroid cancer, Kaposi's sarcoma, Castleman's disease, pancreatic cancer, leukemia, lymphoma, and multiple myeloma.

In another preferred embodiment, the disease associated with the activity or expression of JAK kinase is a disorder associated with JAK1.

In another preferred embodiment, the disorder associated with JAK1 is selected from the group consisting of type 1 diabetes, lupus, multiple sclerosis, rheumatoid arthritis, psoriasis, asthma, atopic dermatitis, autoimmune thyroid disease, ulcerative colitis, Crohn's disease and alopecia areata.

It should be understood that in the present invention, any of the technical features specifically described above and below (such as in the Example) can be combined with each other, thereby constituting new or preferred technical solutions. Limited by space, it will not be repeated herein.

### DETAILED DESCRIPTION OF THE INVENTION

Through extensive and in-depth research, the present inventors have accidentally discovered a new JAK inhibitor for the first time, which has a novel structure, and has good biological activity and extremely excellent selectivity. Specifically, the selectivity of the compounds of the present invention represented by the ratio of JAK2/JAK1 or the selectivity represented by the ratio of JAK3/JAK1 is increased by an average of about 12 times. Therefore, the side effects associated with JAK3 inhibition of the compounds of the present invention are extremely significantly reduced, and the safety will be significantly improved. On this basis, the present invention was completed.

### Terms

In the present invention, unless otherwise specified, the terms used have the general meanings known to those skilled in the art.

When a substituent is described by a conventional chemical formula written from left to right, the substituent also includes a chemically equivalent substituent obtained by writing a structural formula from right to left. For example, -CH₂O- is equivalent to -OCH₂-.

As used herein, the term "about" means that the value can change by no more than 1% from the enumerated value when used in reference to a specific enumerated value. For example, as used herein, the expression "about 100" includes 99 and 101 and all values therebetween (e. g., 99.1, 99.2, 99.3, 99.4, etc.).

As used herein, the terms "contain" or "include (comprise)" may be open, semi-closed, and closed. In other words, the term also includes "consisting essentially of" or "consisting of".

As used herein, the term "alkyl" includes a linear or branched alkyl. For example, C₁-C₆ alkyl represents a linear or branched chain alkyl having 1 to 6 carbon atoms, such as methyl, ethyl, propyl, isopropyl, butyl, isobutyl, tert-butyl, and the like. In the present invention, alkyl can be optionally substituted or unsubstituted, and the substituted alkyl includes haloalkyl, benzyl, and the like.

As used herein, the term "alkenyl" includes a linear or branched alkenyl. For example, C₂-C₆ alkenyl refers to a linear or branched chain alkenyl having 2-6 carbon atoms, such as vinyl, allyl, 1-propenyl, isopropenyl, 1-butenyl, 2-butenyl, or the like.

As used herein, the term "alkynyl" includes a straight or branched chain alkynyl. For example, C₂-C₆ alkynyl refers to a linear or branched chain alkynyl with 2-6 carbon atoms, such as acetenyl, propinyl, butynyl, or the like.

As used herein, the term "cycloalkyl" refers to a cyclic alkyl containing a specific number of C atoms, such as "C3-C10 cycloalkyl" refers to cycloalkyl having 3-10 (preferably 3, 4, 5, 6, 7 or 8) carbon atoms. It may be monocyclic, such as cyclopropyl, cyclobutyl, cyclopentyl, cyclohexyl, or the like. It can also be bicyclic, such as a bridged ring or a spiro ring. In the present invention, cycloalkyl is intended to include substituted cycloalkyl.

As used herein, the term "C1-C6 alkoxy" refers to a linear or branched chain alkoxy having 1-6 carbon atoms; which has the formula of C1-C6 alkyl-O- or C1-C5 alkyl-O-C1-CS (e. g., -CH₂-O-CH₂CH₃, -CH₂-O-(CH₂)₂CH₃, -CH₂CH₂-O-CH₂CH₃), such as methoxy, ethoxy, propoxy, isopropoxy, butoxy, isobutoxy, tert-butoxy, etc..

As used herein, "heterocyclyl" refers to a saturated or partially saturated cyclic group having a heteroatom selected from N, S, and O, and "3-10-membered heterocyclyl" refers to a saturated or partially saturated cyclic group having 3-10 atoms wherein 1-3 atoms are heteroatoms selected from N, S, or O. It can be monocyclic, and it can also be bicyclic, such as a bridged ring or a spiro ring. The 3-10 membered heterocyclyl is preferably 3-8 membered heterocyclyl, more preferably 3-6 membered heterocyclyl, more preferably 6-8 membered heterocyclyl. Specific examples may be oxetanyl, azetidinyl, tetrahydro-2H-pyranyl, piperidinyl, piperazinyl, tetrahydrofuranyl, morpholinyl and pyrrolidinyl, etc.. Heterocycloalkyl or heterocyclyl may be optionally substituted or unsubstituted.

As used herein, "aryl" refers to an aromatic ring group having no heteroatoms on the ring, and "C6-C12 aryl" refers to an aromatic ring group having 6 to 12 carbon atoms without heteroatoms on the ring, the aryl may be fused to heteroaryl, heterocyclyl or cycloalkyl ring, wherein the ring connected to the parent structure is aryl ring. Such as phenyl (i. e. six-membered aromatic ring), naphthyl, etc., the six-membered aryl is also intended to contain six-membered aryl fused 5-6-membered cycloalkyl and six-membered aryl fused 5-6-membered heterocycloalkyl. C6-C12 aryl is preferably C6-C10 aryl. Aryl can be optionally substituted or unsubstituted.

As used herein, "heteroaryl" refers to a cyclic aromatic group having 1-3 atoms selected from N, S, and O, and "5-12 membered heteroaryl" refers to a cyclic aromatic group having 5-12 atoms wherein 1-3 atoms are heteroatoms selected from N, S, and O. It can be monocyclic, and it can also be fused. Specific examples may be pyridyl, pyridazinyl, pyrimidinyl, pyrazinyl, triazinyl, pyrrolyl, pyrazolyl, imidazolyl, (1,2,3)-triazolyl, and (1,2,4)-triazolyl, tetrazolyl, furyl, thienyl, isoxazolyl, thiazolyl, oxazolyl, etc.. The heteroaryl ring may be fused to aryl, heterocyclyl or cycloalkyl, and the ring attached to the parent structure is heteroaryl. Heteroaryl can be optionally substituted or unsubstituted. When being substituted, the substituents are preferably one or more of the following groups independently selected from alkyl, deuterated alkyl, haloalkyl, alkoxy, haloalkoxy, alkenyl, alkynyl, alkylthio, alkylamino, halogen, amino, nitro, hydroxyl, sulfydryl, cyano, cycloalkyl, heterocyclyl, aryl, heteroaryl, cycloalkylthio, oxo, amido, sulfonamido, formyl, formamido, carboxyl and carboxylate, etc..

As used herein, "halogen" or "halogen atom" refers to F, Cl, Br, and I. More preferably, the halogen or halogen atom is selected from F, Cl and Br.

In the present invention, the term "amido" refers to a group with the structure of -CONRR', wherein R and R' can independently represent hydrogen, alkyl, cycloalkyl, aryl, heterocyclyl, as defined above. R and R' can be the same or different in dialkylamine fragments.

In the present invention, the term "sulfonamido" refers to a group with the structure of -SO₂NRR', wherein R and R' can independently represent hydrogen, alkyl, cycloalkyl, aryl, heterocyclyl, as defined above. R and R' can be the same or different in dialkylamine fragments.

In the present invention, the term "formyl" refers to a group comprising -CHO.

In the present invention, the term "formamido" refers to a group containing 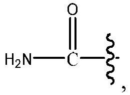 and the formamido is also intended to comprise substituted formamido having the formula of 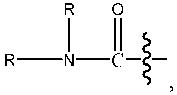 wherein each R independently represents hydrogen, alkyl, cycloalkyl, cycloalkenyl, aryl, heteroaryl, heterocyclyl, as defined above. Each R may be the same or different.

In the present invention, "amino" refers to a group with the structure of -N-RR' , R and R' each independently represents hydrogen, alkyl, cycloalkyl, aryl, heteroaryl, heterocyclyl, as defined above, R and R' may be the same or different.

In the present invention, "sulfinyl" refers to a group with the structure of -S(O)-R, R independently represents hydrogen, alkyl, cycloalkyl, aryl, heteroaryl, heterocyclyl, as defined above.

In the present invention, "sulfonyl" refers to a group with the structure of -S(O)₂-R, R independently represents hydrogen, alkyl, cycloalkyl, aryl, heteroaryl, heterocyclyl, as defined above.

As used herein, an "ester group" refers to -C (O)-O-R or R-C (O)-O-, wherein R independently represents hydrogen, alkyl, cycloalkyl, aryl, heteroaryl, heterocyclyl with the definitions as described above.

In the present invention, the term "substituted" refers to one or more hydrogen atoms on a specific group being substituted by a specific substituent. The specific substituents are those described in the preceding paragraph or those present in each Example. Unless otherwise specified, a substituted group may have a substituent selected from a specific group at any substitutable position of the group, and the substituent may be the same or different in each position. Those skilled in the art should understand that the combinations of substituents contemplated by the present invention are those that are stable or chemically achievable.

Unless specifically indicated as "substituted or unsubstituted", the groups of the present invention may be substituted by substituents selected from the group consisting of deuterium, halogen, cyano, nitro, hydroxyl, amino, C1-C6 alkyl, C2-C6 alkenyl, C2-C6 alkynyl, C1-C6 alkoxy, 3-10-membered heterocycloalkyl, C3-C10 cycloalkyl, 5-12-membered heteroaryl, and C6-C12 aryl.

In the present invention, the term "more" independently refers to 2, 3, 4 or 5.

Unless otherwise specified, the structural formula described in the present invention is intended to include all isomeric forms (e. g., enantiomeric, diastereomeric, and geometric (or conformational) isomers): for example, R and S configurations of asymmetric centers, (Z) and (E) isomers of double bonds, etc. Thus, a single stereochemical isomer of the compound of the invention or a mixture of its enantiomers, diastereomers or geometric isomers (or
conformational isomers) is within the scope of the invention.

As used herein, the term "tautomer" means that structural isomers with different energies can cross a low energy barrier and thus convert to each other. For example, proton tautomers (i.e. proton shift) include intertransformation through proton migration, such as 1H-indazole and 2H-indazole. Valence tautomers include interchange through some bonding electron recombination.

As used herein, the term "solvate" refers to a complex with specific proportion formed by the compound of the invention coordinates with a solvent molecule.

### Active ingredient

As used herein, "the compound of the present invention" refers to the compound represented by the formula I, and further comprises the stereoisomer or optical isomer, pharmaceutically acceptable salt, prodrug or solvate of the compound of formula I.

The compound of formula I has the following structure: 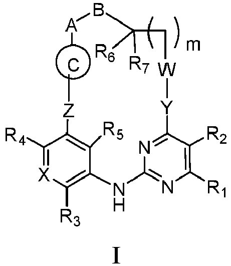 wherein, A, B, C, W, X, Y, Z, R₁, R₂, R₃, R₄, R₅, R₆, R₇, and m are defined as above.

Preferably, the compound of formula I has the structure shown in formula II: 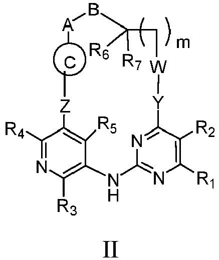 wherein, A, B, C, W, Y, Z, R₁, R₂, R₃, R₄, R₅, R₆, R₇, and m are defined as above. Preferably, in the above-mentioned formulas, Z is a bond.

Preferably, in the above-mentioned formulas, Y is NH.

Preferably, the compound of formula I has the structure shown in formula III: 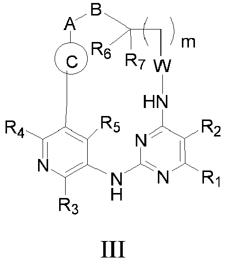 wherein, A, B, C, W, R₁, R₂, R₃, R₄, R₅, R₆, R₇, and m are defined as above.

Preferably, in the above-mentioned formulas, ring C is independently selected from the substituted or unsubstituted group consisting of 6 membered heterocycloalkyl, C6 cycloalkyl, 6 membered heteroaryl, and C6 aryl; wherein, the "substituted" refers to being substituted by one or more groups selected from the group consisting of D, halogen, amino, nitro, hydroxyl, sulfydryl, cyano, carboxyl, sulfydryl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl.

Preferably, in the above-mentioned formulas, ring C is selected from the group consisting of 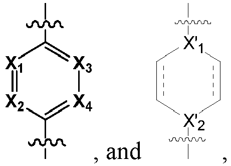
wherein, -̅-̅-̅-̅-̅ represents single bond or double bond;
X₁, X₂, X₃, X₄, X'₁ and X'₂ are each independently selected from the group consisting of N, and C-R_{d}; and 0, 1, 2 or 3 of X₁, X₂, X₃, and X₄ are N;
R_{d} is defined as above.

Preferably, in the above-mentioned formulas, W is independently selected from the group consisting of C(R_{c})₂, 6 membered heterocycloalkyl, C6 cycloalkyl, 6 membered aryl or heteroaryl, and Lᵢ-(CH₂)ₙ-L₂; preferably, W is independently 6 membered aryl or heteroaryl; wherein, L₁ is independently selected from the group consisting of absent, 6 membered heterocycloalkyl, C6 cycloalkyl, and 6 membered aryl or heteroaryl, L₂ is independently selected from the group consisting of N-R_{b}, C(= O)O-, O, S, SO, and SO₂;
the above heterocycloalkyl, cycloalkyl, heteroaryl, and aryl may be further optionally substituted by one or more Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
R_{b}, R_{c}, and n are defined as above.

Preferably, in the above-mentioned formulas, R₃ is halogen.

Preferably, in the above-mentioned formulas, A is C(=O).

Preferably, in the above-mentioned formulas, A is N-R_{b}; wherein, R_{b} is independently selected from the group consisting of H, and C1-C6 alkyl.

Preferably, in the above-mentioned formulas, A is -C(= O)NH-, and B is bond.

Preferably, in the above-mentioned formulas, m is 1, 2, 3, or 4.

As used herein, "pharmaceutically acceptable salt" refers to a salt formed by a compound of the present invention and an acid or a base suitable for use as a medicine. Pharmaceutically acceptable salt comprises inorganic salt and organic salt. A preferred class of salt is a salt formed by a compound of the present invention with an acid. The acids suitable for salt formation include but are not limited to mineral acids such as hydrochloric acid, hydrobromic acid, hydrofluoric acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, phenylmethanesulfonic acid, and benzenesulfonic acid; and acidic amino acids such as aspartic acid and glutamic acid.

### Preparation method of compound

Methods for preparing compounds of formula I are described in the following schemes and examples. Raw materials and intermediates are purchased from commercial sources, prepared by known steps, or otherwise described. In some cases, the sequence of steps to perform the reaction scheme may be changed to facilitate the reaction or avoid unwanted side reaction products.

Generally, in the preparation process, each reaction is usually carried out in an inert solvent at room temperature to reflux temperature (e. g., 0°C to 150°C, preferably 10°C to 100°C). The reaction time is usually 0.1-60 hours, preferably 0.5-48 hours.

Preferably, the compound of the present invention can be prepared with the following steps
in an inert solvent, compound A₈ is reacted in the presence of a catalyst to obtain compound I. 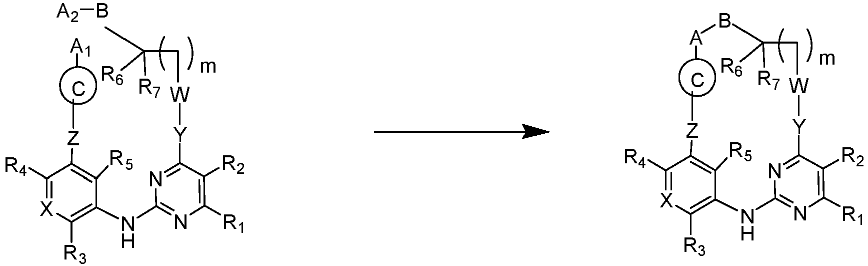 wherein,
A₁ is selected from the group consisting of carboxyl, sulfonic group, CO-O-R", and -CO-NH-R"; wherein, R" is selected from the substituted or unsubstituted group consisting of C1-C6 alkyl, C2-C6 alkenyl, C2-C6 alkynyl, 3-10 membered heterocyclyl, C3-C10 cycloalkyl, 5-12 membered heteroaryl, and C6-C12 aryl;
the "substituted" refers to being substituted by one or more groups selected from the group consisting of D, halogen, amino, amine, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocyclyl, C3-C10 cycloalkyl, 5-12 membered heteroaryl, and C6-C12 aryl;
A₂ is selected from the group consisting of amino and hydroxyl;
A, B, C, W, X, Y, Z, R₁, R₂, R₃, R₄, R₅, R₆, R₇ and m are defined as above.

The starting materials and reagents used in the compound synthesis method of the invention can be commercially purchased or synthesized by the method reported in the literature.

### Pharmaceutical composition and method of administration

Since the compounds of the present invention have excellent JAK kinase inhibitory activity, the compound of the present invention or the stereoisomer or optical isomer, pharmaceutically acceptable salt, prodrug or solvate thereof, and the pharmaceutical composition containing the compound of the present invention as the main active ingredient can be used to prevent and/or treat (stabilize, mitigate or cure) JAK kinase-related diseases (e. g, skin diseases, rheumatoid arthritis, multiple sclerosis, type I diabetes, psoriatic arthritis, juvenile arthritis, Crohn's disease, myasthenia gravis, cancer (including prostate cancer, kidney cancer, liver cancer, breast cancer, lung cancer, thyroid cancer, Kaposi's sarcoma, Castleman's disease, pancreatic cancer, leukemia, lymphoma or multiple myeloma, etc.)).

The pharmaceutical composition of the present invention comprises a safe and effective amount of the compound of the present invention and a pharmaceutically acceptable excipient or carrier, wherein "safe and effective amount" refers to the amount of compound is sufficient to significantly improve the condition, not to produce severe side effects. Typically, the pharmaceutical composition contains 1-2000 mg of the compound of the present invention / dosage, and preferrably contains 10-200mg of the compound of the present invention / dosage. Preferably, "one dosage" is a capsule or a pill.

"Pharmaceutically acceptable carrier" refers to one or more compatible solid or liquid filler or gel substances, which are suitable for human use, and must be sufficiently pure and sufficiently low toxicity. "Compatible" herein refers to each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween^{®}), wetting agent (such as lauryl sodium sulfate), colorant, flavoring, stabilizer, antioxidant, preservative, pyrogen-free water, etc..

There is no special limitation of administration mode for the compound or pharmaceutical compositions of the present invention, and the representative administration mode includes (but is not limited to) oral, parenteral (intravenous, intramuscular or subcutaneous).

Solid dosage forms for oral administration include capsules, tablets, pills, powders and granules. In these solid dosage forms, the active compounds are mixed with at least one conventional inert excipient (or carrier), such as sodium citrate or dicalcium phosphate, or mixed with any of the following components: (a) fillers or compatibilizer,such as starch, lactose, sucrose, glucose, mannitol and silicic acid; (b) binders, such as hydroxymethyl cellulose, alginate, gelatin, polyvinylpyrrolidone, sucrose and arabic gum; (c) humectant, such as, glycerol; (d) disintegrating agent, such as agar, calcium carbonate, potato starch or tapioca starch, alginic acid, certain composite silicates, and sodium carbonate; (e) dissolution-retarding agents, such as paraffin; (f) absorption accelerators, such as quaternary ammonium compounds; (g) wetting agents, such as cetyl alcohol and glyceryl monostearate; (h) adsorbents, for example, kaolin; and (i) lubricants such as talc, calcium stearate, magnesium stearate, solid polyethylene glycol, lauryl sodium sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, such as ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or the combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

The compounds of the present invention can be administered alone or in combination with other pharmaceutically acceptable compounds (such as JAK inhibitors).

In combination administration, the pharmaceutical composition further comprises one or more (2, 3, 4, or more) other pharmaceutically acceptable compounds (e. g., JAK inhibitors). The one or more (2, 3, 4, or more) of the other pharmaceutically acceptable compounds (e. g., JAK inhibitors) may be used simultaneously, separately, or sequentially with the compounds of the present invention for the prevention and/or treatment of diseases related to the activity or expression of JAK kinase.

When the pharmaceutical composition is used, a safe and effective amount of the compound of the present invention is applied to a mammal (such as a human) in need of treatment, wherein the dose is considered as a pharmaceutically effective dose. For a person weighing 60kg, the daily dose is usually 1 to 2000mg, preferably 20 to 500mg. Of course, the particular dose should also depend on various factors, such as the route of administration, patient healthy status, which are well within the skills of an experienced physician..

### The main advantages of the present invention:

1. The compound of the invention has a novel structure and excellent JAK kinase inhibitory effect;
2. The compound of the present invention can be used as a JAK kinase inhibitor, especially as a highly selective inhibitor of JAK1.

The present invention will be further illustrated below with reference to the specific examples. It should be understood that these examples are only to illustrate the invention but not to limit the scope of the invention. The experimental methods without specific conditions in the following examples usually follow conventional conditions, or according to the conditions recommended by the manufacturer. Unless indicated otherwise, percentage and parts are calculated by weight.

Unless otherwise specified, the experimental materials and reagents used in the following examples can be obtained from commercial sources.

### Example

### General materials and testing methods:

Methods for the synthesis of the compounds of the present invention are shown in the following schemes, methods and examples. The starting materials are commercially available or can be prepared according to known methods in the art or described herein. The compounds of the present invention can be illustrated by the specific examples shown below. However, these specific examples should not be construed as being of the only kind of the present invention. These examples further detail the preparation of the compounds of the invention. Those skilled in the art will easily understand that known changes in conditions and processes can be used to prepare these compounds. Unless otherwise stated, all temperatures are in degrees Celsius.

Thin layer chromatography (PTLC) was prepared on a 20 × 20cm plate (500 micron of silica gel). Biotage rapid chromatography system was used for silica gel chromatography.

1H NMR was conducted by Bruker AscendTM400 spectrometer at 400MHz at 298°K, and the chemical shifts (ppm) of the residual protons in the deuterated reagent were given as reference;
CHCl3 δ = 7.26 ppm, CH3OH or CH3OD δ = 3.30 ppm, DMSO-*d*₆ δ = 2.50 ppm
LCMS chromatography was conducted by Agilent Technology 1200 series or 6120 quadrupole spectrometer. For LC, mobile phase was acetonitrile (A) and water (B) and 0.01% formic acid, eluent gradients: 6.0 min 5-95% A, 5.0 min 60-95% A, 5.0 min 80-100% A and 10 min 85-100% A, and capillary column was SBC1850 mm × 4.6mm × 2.7 micron.

Mass spectrometry (MS) was determined by electrospray ion mass spectrometry (ESI).

HPLC mass spectrometry analysis conditions:
LC1:
   Column: SB-C18 50mm × 4.6mm × 2.7 µm
   Temperature: 50 ° C
   Eluent: 5:95 to 95:5 volume/volume acetonitrile/water + 0.01% formic acid, 6 minutes.
   Flow rate: 1.5 mL/min, injection 5µL
   Detection: PDA, 200-600 nm
   MS: mass range 150-750 amu; Positive ion electrospray ionization
LC2:
   Column: SB-C18 50mm × 4.6mm × 2.7 µm
   Temperature: 50° C
   Eluent: 5:95 to 95:5 volume/volume acetonitrile/water + 0.05% TFA for more than 3.00 minutes.
   Flow rate: 1.5 mL/min, injection 5µL
   Detection: PDA, 200-600 nm
   MS: mass range 150-750 amu; Positive ion electrospray ionization
LC3:
   Column: SB-C18 50mm × 4.6mm × 2.7 µm
   Temperature: 50° C
   Eluent: 10:90 to 98:2 volume/volume acetonitrile/water + 0.05% TFA for more than 3.75 minutes.
   Flow rate: 1.0 mL/min, injection 10 µL
   Detection: PDA, 200-600 nm
   MS: mass range 150-750 amu; Positive ion electrospray ionization

### Abbreviations:

AcOH = acetic acid
Alk is alkyl
AR is aryl
Boc = tert-butoxycarbonyl
bs = broad peak
CH₂Cl₂= dichloromethane
d = doublet
dd = double doublet
DBU = 1,8-diazabicyclo[5.4.0]undec-7-ene
DCM = dichloromethane
DEAD = diethyl azodicarboxylate
DMF = N,N-dimethylformamide
DMSO = dimethyl sulfoxide
EA = ethyl acetate
ESI = electrospray ionization
Et = ethyl
EtOAc = ethyl acetate
EtOH = ethanol
h = hour
HOAc = acetic acid
LiOH = lithium hydroxide
m = multiple
Me = methyl
MeCN = acetonitrile
MeOH = methanol
MgSO₄= magnesium sulfate
min = minute
MS = mass spectrometry
NaCl = sodium chloride
NaOH = sodium hydroxide
Na₂SO₄= sodium sulfate
NMR = nuclear magnetic resonance spectrum
PE = petroleum ether
PG = protecting group
Ph = phenyl
rt = room temperature
s = singlet
t = triplet
TFA = trifluoroacetic acid
THF = tetrahydrofuran
TS = p-toluenesulfonyl (toluenesulfonyl)

### Example 1 The synthetic route of A1:

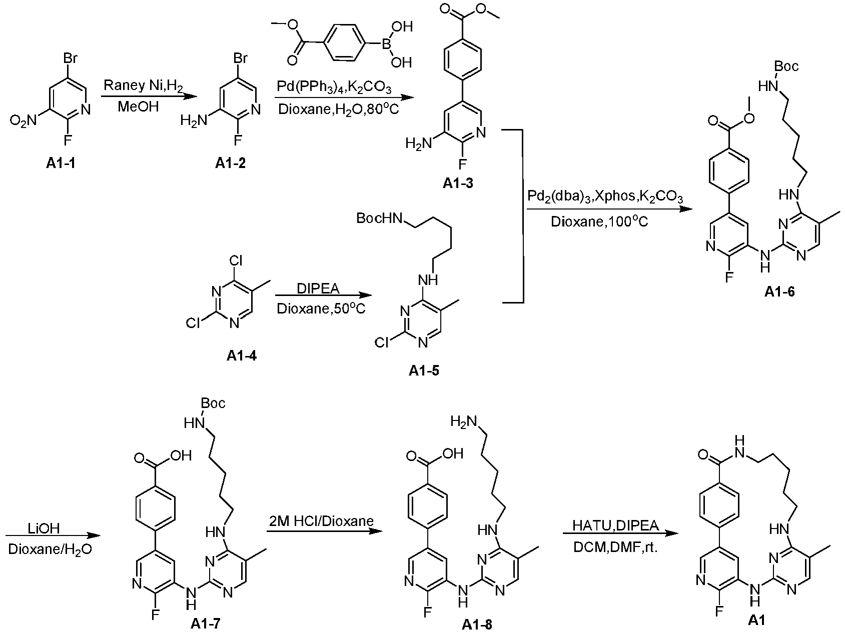

### Step 1: 3-Amino-5-bromo-2-fluoropyridine (A1-2)

5-Bromo-2-fluoro-3-nitropyridine (A1-1, 3.00g, 13.6mmol) was added to methanol (30ml), stirred at room temperature for 5min, raney nickel (150mg) was added, and hydrogen was introduced into the reaction system for 6h. After TLC confirmed that the reaction was completed, the reaction solution was filtered, and methanol was concentrated. The residue was purified by column chromatography to obtain 3-amino-5-bromo-2-fluoropyridine (**A1-2**, 1.879g, yield: 72.5%) as a light yellow solid.

### Step 2: Methyl 4-(5-amino-6-fluoropyridin-3-) benzoate (A1-3)

Under nitrogen protection, 3-amino-5-bromo-2-fluoropyridine **(A1 -2, 300**mg, 1.57mmol), p-methoxycarbonylphenylboronic acid (282.7mg,1.57mmol) and potassium carbonate (542.7mg,3.93mmol) were added into 1,4-dioxane (12ml) and water (3ml), stirred for 5min, then tetrakis(triphenylphosphine)palladium (54.5mg, 0.03mmol) was added, and the temperature was raised to 80°C to stir for 4 hours. After TLC confirmed that the reaction was completed, 1, 4-dioxane was concentrated, then EA and deionized water were added, and the organic layer was extracted and collected, dried over anhydrous sodium sulfate. EA was concentrated, and the residue was purified by column chromatography to obtain methyl 4-(5-amino-6-fluoropyridin-3-) benzoate **(A1-3,** 368.2mg, yield: 95.2%) as a white-like solid. ¹H NMR (400 MHz, CDCl₃) δ 8.15 (t, *J =* 8.3 Hz, 2H), 7.81 (s, 1H), 7.72 (d, *J =* 8.3 Hz, 1H), 7.61 (d, *J =* 8.3 Hz, 1H), 7.33 (dd, J=10.2, 2.0 Hz, 1H), 3.98 (d, *J =* 2.0 Hz, 3H).

### Step 3: Tert-butyl 5-((2-chloro-5-methylpyrimidin-4-) amino) amylamine formate (A1-5) 2,4-dichloro-5-methylpyrimidine (A1 -4, 300mg, 1.84mmol),

N-tert-butoxycarbonylcadaverine (744.6mg, 3.68mmol) and N,N-diisopropylethylamine (475.6mg, 3.68mmol) were added to 1,4-dioxane (3ml), heated to 50°C, and stirred for 16h. 1,4-Dioxane was concentrated, then EA and deionized water were added. The mixture was extracted and delaminated, and the organic layers were collected, dried over anhydrous sodium sulfate. EA was concentrated, and the residue was purified by column chromatography to obtain tert-butyl 5-((2-chloro-5-methylpyrimidin-4-) amino) amylamine formate (**A1-5**, 595.5mg, yield: 98.4%) as a yellowish oil. ¹H NMR (400 MHz, CDCl₃) δ 7.79 (s, 1H), 3.51 (dd, *J* = 12.9, 6.6 Hz, 2H), 3.13 (d, *J* = 6.2 Hz, 2H), 2.00 (s, 3H), 1.71 - 1.61 (m, 2H), 1.54 (dt, *J* = 14.5, 7.0 Hz, 2H), 1.43 (m, 11H).

### Step 4: Methyl 4-(5-((4-(5-tert-butoxycarbonylamino) pentyl)-5-methylpyrimidin-2-) amino) -6-fluoropyridin-3-) benzoate (A1-6)

Under nitrogen protection, methyl 4-(5-amino-6-fluoropyridin-3-) benzoate **(A1-3,** 141mg, 0.57mmol), tert-butyl 5-((2-chloro-5-methylpyrimidin-4-) amino) amylamine formate (**A1-5**, 179mg, 0.55mmol) and potassium carbonate (150mg, 1.1 mmol) were added to 1,4-dioxane (10ml), and stirred for 5min. Then tris(dibenzylideneacetone)dipalladium (50mg,0.05mmol) and 2-dicyclohexylphosphino-2',4',6'-triisopropylbiphenyl (52mg,0.11mmol) were added to the mixture at one time, and the mixture was heated to 100°C and stirred for 5h. After TLC confirmed that the reaction was completed, 1, 4-dioxane was concentrated, then EA and deionized water were added. The organic layer was extracted and collected, dried over anhydrous sodium sulfate. EA was concentrated, and the residue was purified by column chromatography to obtain methyl 4-(5-((4-(5-tert-butoxycarbonylamino) pentyl)-5-methylpyrimidin-2-) amino) -6-fluoropyridin-3-) benzoate (**A1-6**, 150mg, yield: 48.7%) as a white solid. ¹H NMR (400 MHz, CDCl₃) δ 9.35 (d, *J* = 9.7 Hz, 1H), 8.15 (d, *J* = 7.9 Hz, 2H), 7.95 (s, 1H), 7.80 (s, 1H), 7.69 (d, *J* = 7.9 Hz, 2H), 3.98 (s, 3H), 3.53 (dd, *J* = 12.8, 6.4 Hz, 2H), 3.11 (d, *J =* 6.1 Hz, 2H), 2.01 (s, 3H), 1.45 (s, 10H), 1.39 - 1.26 (m, 5H).

### Step 5: 4-(5-((4-(5-tert-butoxycarbonylamino) pentyl -5-methylpyrimidin-2-)amino)-6-fluoropyridin-3-) benzoic acid (A1-7)

Methyl 4-(5-((4-(5-tert-butoxycarbonylamino) pentyl)-5-methylpyrimidin-2-) amino) -6-fluoropyridin-3-) benzoate**(A1-6,** 150mg, 0.28mmol) was added to tetrahydrofuran (9ml) and water (3ml), then lithium hydroxide monohydrate (58mg,1.4mmol) was added, and stirred at room temperature for 16h. After TLC confirmed that the reaction was completed, 1mol/L HCl was added dropwise to adjust pH = 5-6, then tetrahydrofuran was concentrated. Deionized water was added to the reaction solutiom, filtered, and the filter cake was collected, and dried under reduced pressure to obtain 4-(5-((4-(5-tert-butoxycarbonylamino) pentyl-5-methylpyrimidin-2-) amino) -6-fluoropyridin-3-) benzoic acid **(A1-7,** 127mg, yield: 87%) as a white-like solid.

### Step 6: 4-(5-((4-(5-aminopentylamine)-5-methylpyrimidin-2-) amino) -6-fluoropyridin-3-) benzoic acid (A1-8)

4-(5-((4-(5-tert-butoxycarbonylamino) pentyl -5-methylpyrimidin-2-) amino)-6-fluoropyridin-3-) benzoic acid **(A1-7,** 127mg, 0.24mmol) was added to 1,4-dioxane (2ml), and a solution (2ml) of 4mol/L HCl in dioxane was added dropwise, and stirred at room temperature for 2h. After LCMS confirmed that the reaction was completed, the reaction solution was directly concentrated to remove 1,4-dioxane to obtain 4-(5-((4-(5-aminopentylamino)-5-methylpyrimidin-2-) amino)-6-fluoropyridin-3-) benzoic acid **(A1-8,** 114mg, crude) as a white-like solid, which was used directly for the next step without purification.

### Step 7: 2⁶-fluoro-4⁵-methyl-3,5,11-triaza-4 (2,4)-pyrimidina -2(3,5)-pyridina -1(1,4)-benzenacyclododecaphan-12-one (A1)

2-(7-Azabenzotriazol-1-yl)-N,N,N',N'-tetramethyluronium hexafluorophosphate (95mg, 0.25mmol) and N,N-diisopropylethylamine (161.6mg, 1.25mmol) were added to DCM and stirred for 5min. A mixed solution of 4-(5-((4-(5-aminopentylamino)-5-methylpyrimidin-2-) amino)-6-fluoropyridin-3-) benzoic acid **(A1-8,** 53mg, 0.125mmol) in DCM(10ml) and N,N-dimethylformamide (1ml) was slowly added, and stirred at room temperature for 4 hours after the addition. After LCMS confirmed that the reaction was completed, deionized water was added to quench the reaction. The reaction solution was extracted and delaminated, and the organic layer was collected. DCM was concentrated, the residue was purified by reverse phase column, and lyophilized to obtain **A1**(2.3mg, yield: 4.5%) as a white solid. MS (ESI) m/z: calcd 407.20 (M + H), found 407.36; ¹H NMR (400 MHz, DMSO-_{d6}) δ 8.90 (s, 1H), 8.74 (s, 1H), 7.96 (d, *J* = 27.7 Hz, 2H), 7.67 (d, *J =* 31.8 Hz, 3H), 7.47 (s, 2H), 6.48 (s, 1H), 2.88 (s, 2H), 2.74 (s, 2H), 1.90 (s, 3H), 1.37-1.18 (m, 6H).

Examples 2-48 were obtained by referring to the experimental steps of Example 1 and using different starting materials, as shown in Table 1 below.

**Table 1**

| **Example** | Structure | MS (calcd) [M + H]⁺ /MS (found) | Name |
|---|---|---|---|
| **2** | 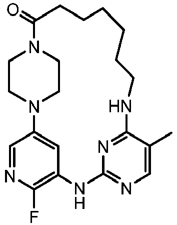 | 414.24/414.49 | 2⁶-fluoro-4⁵-methyl-3,5-diaza-4(2, 4)-pyrimidina -1(1,4)-piperazina-2(3,5)-pyridinac yclododecaphan-12-one |
| **3** | 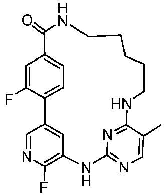 | 425.19/425.10 | 1²,2⁶-difluoro-4⁵-methyl-3,5,11-tri aza-4(2,4)-pyrimidina-2(3,5)-pyrid ina-1(1,4)-benzenacyclododecapha n-12-one |
| **4** | 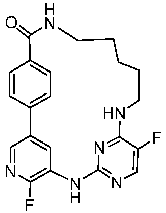 | 411.17/411.40 | 2⁶,4⁵-difluoro-3,5,11-triaza-4(2,4)-pyrimidina-2(3,5)-pyridina-1(1,4)-benzenacyclododecaphan-12-one |
| **5** | 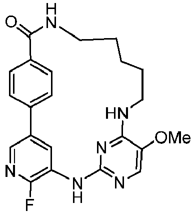 | 423.19/423.13 | 2⁶-fluoro-4⁵-methoxy-3,5,11-triaza -4(2,4)-pyrimidina-2(3,5)-pyridina -1(1,4)-benzenacyclododecaphan-1 2-one |
| **6** | 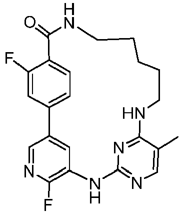 | 425.19/425.29 | 1³,2⁶-difluoro-4⁵-methyl-3,5,11-tri aza-4(2,4)-pyrimidina-2(3,5)-pyrid ina-1(1,4)-benzenacyclododecapha n-12-one |
| **7** | 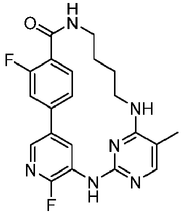 | 411.17/411.21 | 1²,2⁶-difluoro-4⁵-methyl-3,5,10-tri aza-4(2,4)-pyrimidina-2(3,5)-pyrid ina-1(1,4)-benzenacycloundecapha n-11-one |
| **8** | 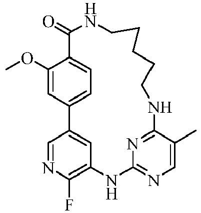 | 437.21/437.16 | 2⁶-fluoro-1³-methoxy-4⁵-methyl-3, 5,1 1-triaza-4(2,4)-pyrimidina-2(3, 5)-pyridina-1(1,4)-benzenacyclodo decaphan-12-one |
| **9** | 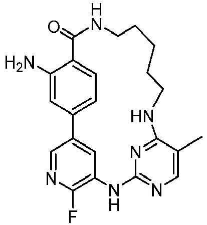 | 422.21/422.47 | 1³-amino-2⁶-difluoro-4⁵-methyl-3, 5,11-triaza-4(2,4)-pyrimidina-2(3, 5)-pyridina-1(1,4)-benzenacyclodo decaphan-12-one |
| **10** | 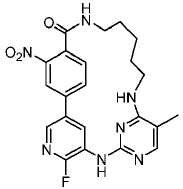 | 452.18/452.45 | 2⁶-fluoro-4⁵-methyl-1³-nitro -3,5,11-triaza-4(2,4)-pyrimidina-2( 3,5)-pyridina-1(1,4)-benzenacyclo dodecaphan-12-one |
| **11** | 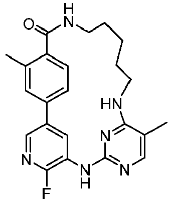 | 421.22/421.41 | 2⁶-fluoro-1³,4⁵-dimethyl-3,5,11-tri aza-4(2,4)-pyrimidina-2(3,5)-pyrid ina-1(1,4)-benzenacyclododecapha n-12-one |
| **12** | 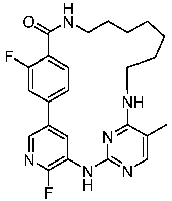 | 453.22/453.15 | 1³,2⁶-difluoro-4⁵-methyl -3,5,13-triaza-4(2,4)-pyrimidina-2( 3,5)-pyridina-1(1,4)-benzenacyclo dodecaphan-12-one |
| **13** | 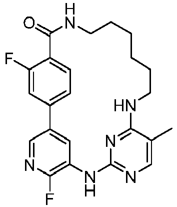 | 439.21/439.25 | 1³, 2⁶-difluoro-4⁵-methyl -3,5,12-triaza-4(2,4)-pyrimidina-2( 3,5)-pyridina-1(1,4)-benzenacyclot ridecaphan-13-one |
| **14** | 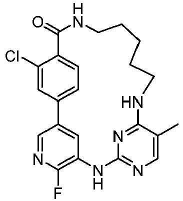 | 441.16/441.37 | 1³-chloro-2⁶-fluoro-4⁵-methyl-3,5, 11-triaza-4(2,4)-pyrimidina-2(3, 5)-pyridina-1(1,4)-benzenacyclodode caphan-12-one |
| **15** | 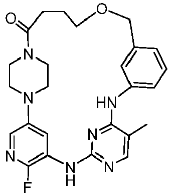 | 478.24/478.20 | 2⁶-fluoro-4⁵-methyl -8-oxa-3,5-diaza-4(2,4)-pyrimidina -1(1,4)-piperazina-2(3,5)-pyridina-6(1,3)-benzenacyclododecaphan-1 2-one |
| **16** | 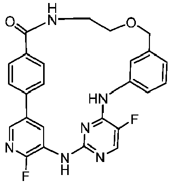 | 475.17/475.10 | 2⁶,4⁵-difluoro -oxa-3, 5,11-triaza-4(2,4)-pyrimidi na-2(3,5)-pyridina-1(1,4),6(1,3)-di benzenacyclododecaphan-12-one |
| **17** | 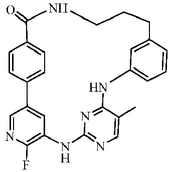 | 455.20/455.10 | 2⁶-fluoro-4⁵-methyl -3,5,10-triaza-4(2,4)-pyrimidina-2( 3,5)-pyridina-1(1,4),6(1,3)-dibenze nacycloundecaphan-11-one |
| **18** | 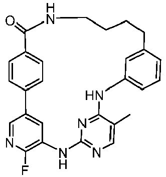 | 469.22/469.47 | 2⁶-fluoro-4⁵-methyl -3,5,11-triaza-4(2,4)-pyrimidina-2( 3,5)-pyridina-1(1,4),6(1,3)-dibenze nacyclododecaphan-12-one |
| **19** | 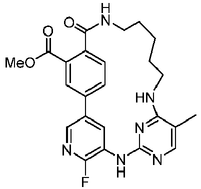 | 465.21/465.47 | methyl 2⁶-fluoro-4⁵-methyl-12-oxo-3,5,11 -triaza-4(2,4)-pyrimidina-2(3,5)-py ridina-1(1,4)-benzenacyclododeca phane-1³-carboxylate |
| **20** | 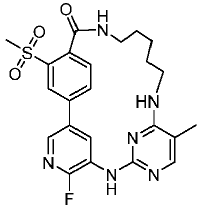 | 485.18/485.55 | 2⁶-fluoro-4⁵-methyl-1³-methylsulf onyl-3,5,11-triaza-4(2,4)-pyrimidi na-2(3,5)-pyridina-1(1,4)-benzena cyclododecaphan-12-one |
| **21** | 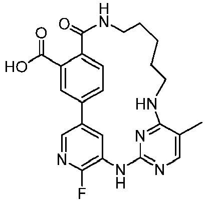 | 451.19/451.23 | 2⁶-fluoro-4⁵-methyl-12-oxo-3, 5,11-triaza-4(2,4)-pyrimidina-2(3, 5)-pyridina-1(1,4)-benzenacyclodo decaphane-12-one-1³-carboxylic acid |
| **22** | 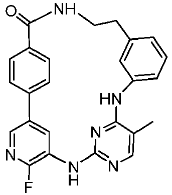 | 441.18/441.25 | 2⁶-fluoro-4⁵-methyl -3,5,9-triaza-4(2,4)-pyrimidina-2(3 ,5)-pyridina-1(1,4),6(1,3)-dibenzen acyclodecaphan-10-one |
| **23** | 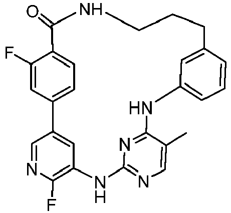 | 473.19/473.20 | 1³,2⁶-difluoro-4⁵-methyl-3,5,10-tri aza-4(2,4)-pyrimidina-2(3,5)-pyrid ina-1(1,4),6(1,3)-dibenzenacyclou ndecaphan-11-one |
| **24** | 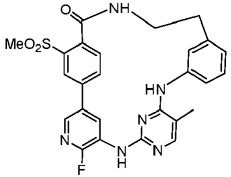 | 519.16/519.27 | 2⁶-fluoro-4⁵-methyl -1³-(methylsulfonyl)-3,5,9-triaza-4 (2,4)-pyrimidina-2(3, 5)-pyridina-1 (1,4),6(1,3)-dibenzenacyclodecaph an-10-one |
| **25** | 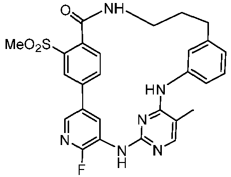 | 533.18/533.10 | 2⁶-fluoro-4⁵-methyl -1³ -(methylsulfonyl)-3,5,10-triaza-4(2,4)-pyrimidina-2(3,5)-pyridina-1(1,4),6(1,3)-dibenzenacycloundec aphan-11-one |
| **26** | 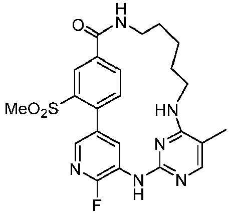 | 485.18/485.10 | 2⁶-fluoro-4⁵-methyl-1²-(methylsulf onyl)-3,5,11-triaza-4(2,4)-pyrimidi na-2(3,5)-pyridina-1(1,4)-benzena cyclododecaphan-12-one |
| **27** | 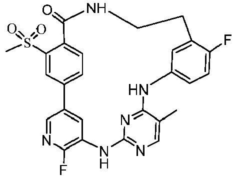 | 537.15/537.20 | 2⁶,6⁴-difluoro-4⁵-methyl-1³(methyl sulfonyl)-3,5,9-triaza-4(2,4)-pyrim idina-2(3,5)-pyridina-1(1,4),6(1,3) -dibenzenacyclodecaphan-10-one |
| **28** | 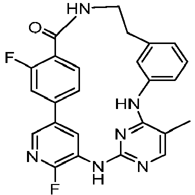 | 459.17/459.23 | 1³,2⁶-difluoro-4⁵-methyl-3,5,9-tria za-4(2,4)-pyrimidina-2(3,5)-pyridi na-1(1,4),6(1,3)-dibenzenacyclode caphan-10-one |
| **29** | 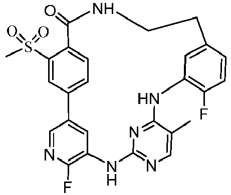 | 537.15/537.20 | 2⁶,6⁶-difluoro-4⁵-methyl-1³(methyl sulfonyl)-3,5,9-triaza-4(2,4)-pyrim idina-2(3,5)-pyridina-1(1,4),6(1,3) -dibenzenacyclodecaphan-10-one |
| 30 | 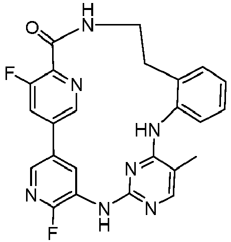 | 460.17/459.70 | 1³,6⁶-difluoro-4⁵-methyl -1³-3,5,9-triaza-4 (2,4)-pyrimidina-1(5,2),2(3,5)-dip yridina -6(1,2)-benzenacyclodecaphan-10-one |
| **31** | 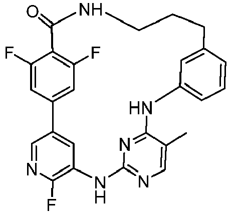 | 491.18/490.10 | 1³,1⁵,2⁶-trifluoro-4⁵-methyl-3,5,10-triaza-4(2,4)-pyrimidina-2(3,5)-pyr idina-1(1,4),6(1,3)-dibenzenacyclo undecaphan-11-one |
| **32** | 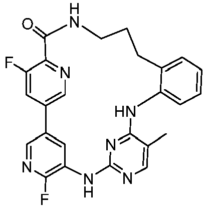 | 474.19/473.40 | 1³,2⁶-difluoro-4⁵-methyl-3,5,10-tri aza-4 (2,4)-pyridina -1(5,2),2(3,5)-dipyridina-6(1,2)-be nzenacycloundecaphan-11-one |
| **33** | 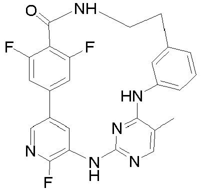 | 477.17/476.60 | 1³,1⁵,2⁶-trifluoro-4⁵-methyl-3,5,9-t riaza-4(2,4)-pyrimidina-2(3,5)-pyri dina-1(1,4),6(1,3)-dibenzenacyclo decaphan-10-one |
| **34** | 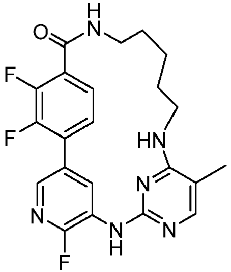 | 443.18/443.30 | 1²,1³,2⁶-trifluoro-4⁵-methyl -3,5,11-triaza-4(2,4)-pyrimidina-2( 3,5)-pyridina-1(1,4)-benzenacyclo dodecaphan-12-one |
| **35** | 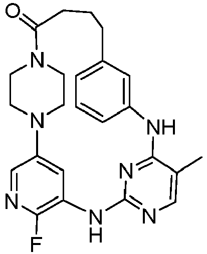 | 448.23/447.90 | 2⁶-fluoro-4⁵-methyl -3, 5-diaza-4(2,4)-pyrimidina -1(1,4)-piperazina-2(3,5)-pyridina-6(1,3)-benzenacyclodecaphan-10-o ne |
| **36** | 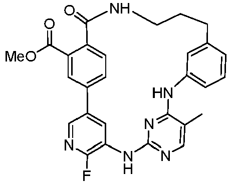 | 513.21/513.20 | Methyl 2⁶-fluoro-4⁵-methyl-11-oxo-3, 5,10-triaza-4 (2,4)-pyrimidina -2(3,5)-pyridina-1(1,4),6(1,3)-dibe nzenacycloundecaphan-1³-carboxy late |
| **37** | 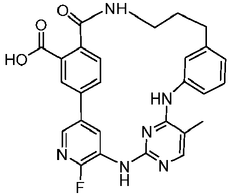 | 499.19/498.31 | 2⁶-fluoro-4⁵-methyl-11-oxo-3,5,10 -triaza-4 (2,4)-pyrimidina -2(3,5)-pyridina-1(1,4),6(1,3)-dibe nzenacycloundecaphan-1³-formic acid |
| **38** | 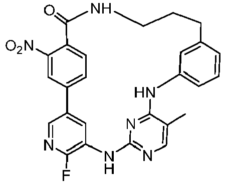 | 500.18/500.20 | 2⁶-fluoro-4⁵-methyl-1³-nitro-3,5,10-triaza-4(2,4)-pyrimidina-2(3,5)-pyri dina-1(1,4),6(1,3)-dibenzenacyclou ndecaphan-11-one |
| **39** | 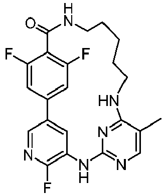 | 443.17/443.10 | 1³,1⁵,2⁶-trifluoro-4⁵-methyl -3,5,11-triaza-4(2,4)-pyrimidina-2( 3,5)-pyridina-1(1,4)-benzenacyclod odecaphan-12-one |
| **40** | 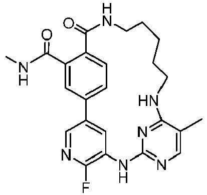 | 464.21/464.20 | 2⁶-fluoro-N,4⁵-dimethyl-12-oxo-3,5 ,11-triaza-4(2,4)-pyrimidina-2(3,5)-pyridina-1(1,4)-benzenacyclododec aphane-1³-formamide |
| **41** | 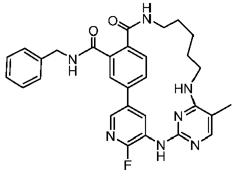 | 540.24/540.30 | N-benzyl-2⁶-fluoro-4⁵-methyl-12-o xo-3, 5,11-triaza-4(2,4)-pyrimidina-2(3,5)-pyridina-1(1,4)-benzenacycl ododecaphane-1³-formamide |
| **42** | 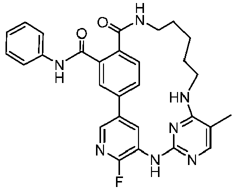 | 526.23/526.30 | 2⁶-fluoro-4⁵-methyl-12-oxo-N-phen yl -3,5,11-triaza-4(2,4)-pyrimidina-2( 3,5)-pyridina-1(1,4)-benzenacyclod odecaphane-1³-formamide |
| **43** | 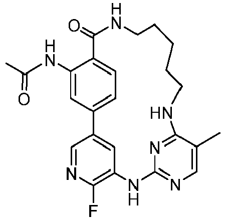 | 464.21/464.20 | N-(2⁶-fluoro-4⁵-methyl-12-oxo-3, 5,11-triaza-4 (2,4)-pyrimidina-2(3,5)-pyridina-1( 1,4)-benzenacyclododecaphane-1³-yl) acetamide |
| **44** | 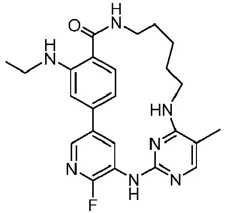 | 450.23/450.30 | 1³-(ethylamino)-2⁶-difluoro-4⁵-met hyl-3,5,11-triaza-4(2,4)-pyrimidina -2(3,5)-pyridina-1(1,4)-benzenacycl ododecaphan-12-one |
| **45** | 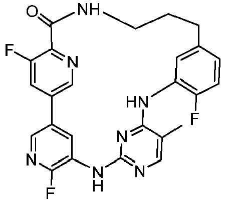 | 492.17/492.30 | 1³,2⁶6⁶-trifluoro-4⁵-methyl-3,5,10-t riaza-4 (2,4)-pyrimidina -1(5,2),2(3,5)-bipyridina-6(1,3)-ben zenacycloundecaphan-11-one |
| **46** | 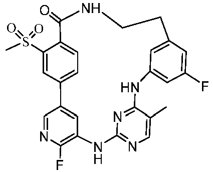 | 537.14/537.20 | 2⁶,6⁵-difluoro-4⁵-methyl-1³(methyls ulfonyl)-3,5,9-triaza-4(2,4)-pyrimid ina-2(3,5)-pyridina-1(1,4),6(1,3)-di benzenacyclodecaphan-10-one |
| **47** | 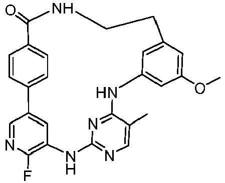 | 471.19/471.50 | 2⁶-fluoro -6⁵-methoxy-4⁵-methyl-3,5, 9-triaza-4(2,4)-pyrimidina-2(3,5)-p yridina-1(1,4),6(1,3)-dibenzenacycl odecaphan-10-one |
| **48** | 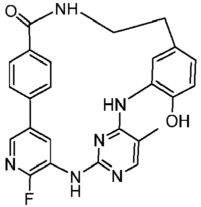 | 457.27/457.10 | 2⁶-fluoro-6⁶-hydroxy-4⁵-methyl-3,5 , 9-triaza-4 (2,4)-pyrimidina -2(3,5)-pyridina-1-2,(1,4),6(1,3)-di benzenacyclodecaphan-10-one |

### Example 49: Biological test

### Biological test method

The activity measurement method of JAK kinase was to use homogeneous time-resolved fluorescence technique. The reaction was performed in 384 shallow well plates with a total reaction volume of 10 µL. The mixture of kinase protein, compound, ATP and substrate was carried out in a reaction buffer of 50 mM Hepes (pH7.0), NaN₃ 0.02%, BSA 0.01%, 0.1mM orthocanadate, 5 mM MgCl₂, and 1 mM DTT. After reacting for 1 hour, antibody capable of recognizing substrate phosphorylation and dye XL-615 and detection buffer (Cisbio) containing EDTA were added to the system. The reaction signal of kinase was detected by PE company's porous plate detector. The parameter settings were excitation light at 320 nm, emission light at 615 nm and 665 nm. The activity of JAK is indirectly reflected by the signal ratio of 665 nm and 615 nm. In the reaction, the background well without enzyme and the total enzyme activity well without compound were set.

The IC₅₀ value of the compound inhibiting protein was obtained by the formula: Y = 100/(1+10^((LogIC50-X)^{∗}HillSlope)).

In JAK1 reaction system, the concentration of ATP was 2 µM, and the concentration of JAK1 protein was 0.2 ng/µL.

In JAK2 reaction system, the concentration of ATP was 2 µM, and the concentration of JAK2 protein was 0.01 ng/µL.

In JAK3 reaction system, the concentration of ATP was 2 µM, and the concentration of JAK3 protein was 0.04 ng/µL.

In TYK2 reaction system, the concentration of ATP was 2 µM and the concentration of TYK2 protein was 0.2 ng/µL.

The test data are divided into the following types: A: IC₅₀ <10 nM; B: IC₅₀ 11-100 nM; C: IC₅₀ 101-1000 nM; D: IC₅₀ 1001-10000 nM; E: IC₅₀ > 10000 nM.

The results are shown in Table 2.

**Table 2**

| Compound No. | JAK 1 activity | JAK 2 activity | JAK 3 activity | TYK 2 activity | Compound No. | JAK 1 activity | JAK 2 activity | JAK 3 activity | TYK 2 activity |
|---|---|---|---|---|---|---|---|---|---|
| **1** | C | D | D | E | **2** | C | C | D | D |
| **3** | B | B | C | D | **4** | D | C | D | E |
| **5** | D | D | D | E | **6** | B | C | C | D |
| **7** | B | C | D | D | **8** | C | D | D | E |
| **9** | C | C | D | D | **10** | B | C | D | D |
| **11** | C | C | D | D | **12** | C | C | D | D |
| **13** | C | C | D | E | **14** | C | C | D | D |
| **15** | B | C | C | D | **16** | B | B | B | D |
| **17** | A | A | B | B | **18** | A | A | A | B |
| **19** | C | C | D | E | **20** | B | D | D | E |
| **21** | C | B | C | D | **22** | A | A | A | B |
| **23** | A | A | C | C | **24** | A | A | B | C |
| **25** | A | A | B | C | **26** | D | D | D | E |
| **27** | B | C | C | E | **28** | A | B | B | D |
| **29** | B | C | D | E | **30** | B | B | C | D |
| **31** | C | C | D | E | **32** | B | B | C | D |
| **33** | C | C | D | E | **34** | C | C | C | E |
| **35** | A | B | C | C | **36** | A | A | B | C |
| **37** | B | B | C | D | **38** | A | A | B | C |
| **39** | D | C | D | E | **40** | C | D | D | E |
| **41** | D | D | D | E | **42** | D | D | D | E |
| **43** | C | C | C | E | **44** | D | D | E | E |
| **45** | B | B | C | D | **46** | A | A | B | C |
| **47** | C | C | D | E | **48** | B | B | C | C |

### Discussion:

The above experimental results suggest that
(1) The compound of formula I of the present invention exhibits very excellent activity of inhibiting JAK, especially against JAK1. The IC50 value of the compound of the present invention can be as low as 10nM or less, so that for subjects weighing about 70kg (such as patients, especially patients with rheumatoid arthritis or psoriasis), daily doses of 10mg to 30mg can be extremely effective in inhibiting JAK, especially JAK1.
(2) The compound of formula I of the present invention exhibits very excellent JAK1 selectivity, i .e. the IC50 ratio of JAK3/JAK1 and/or the IC50 ratio of JAK2/JAK1 is superior to the currently marketed drugs.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula I or a stereoisomer or optical isomer, a pharmaceutically acceptable salt, a prodrug or a solvate thereof, 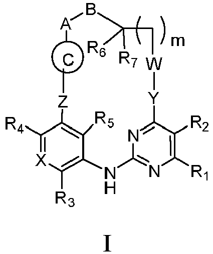 wherein,
A is independently selected from the group consisting of C(=O), -C(=O)O-, C(=O)NH, N-R_{b}, O, S, SO, and SO₂;
B is independently selected from the group consisting of bond, C(=O), N-R_{b}, C(R_{c})₂, C(= O)O-, O, S, SO, and SO₂;
ring C is independently selected from the substituted or unsubstituted group consisting of 3-10 membered heterocycloalkyl, C3-C10 cycloalkyl, 5-12 membered heteroaryl, and C6-C12 aryl;
X is independently selected from the group consisting of N and CR_{d};
Y is independently selected from the group consisting of C(=O), N-R_{b}, C(R_{c})₂, C(=O)O-, O, S, SO, and SO₂;
Z is independently selected from the group consisting of bond, N-R_{b}, O, S, and C(R_{c})₂;
W is independently selected from the group consisting of C(R_{c})₂, 3-10 membered heterocycloalkyl, C3-C10 cycloalkyl, 5-12 membered heteroaryl, C6-C12 aryl, and L₁-(CH₂)ₙ-L₂; wherein L₁ is independently selected from the group consisting of absent, 3-10 membered heterocycloalkyl, C3-C10 cycloalkyl, 5-12 membered heteroaryl, and C6-C12 aryl, L₂ is independently selected from the group consisting of N-R_{b}, C(=O)O-, O, S, SO, and SO₂;
R_{b} is independently selected from the group consisting of H, and C1-C6 alkyl;
R_{c} is each independently selected from the group consisting of H, halogen, amino, nitro, hydroxyl, cyano, carboxyl, sulfonyl, sulfinyl, amido, sulfonamido, ester group, C1-C6 alkyl, C1-C6 alkoxy, 3-10 membered heterocycloalkyl, C3-C10 cycloalkyl, 5-12 membered heteroaryl, and C6-C12 aryl;
R₁, R₂, R₃, R₄, R₅, R₆, R₇ and R_{d} are each independently selected from the group consisting of H, D, halogen, amino, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
or, R₁ and R₂ together with the atoms to which they are attached form the substituted or unsubstituted group consisting of 5-6 membered aryl or heteroaryl, 3-10 membered heterocyclyl, and C3-C10 cycloalkyl;
the H atom in -(CH₂)ₘ- and -(CH₂)ₙ- can be optionally substituted by one or more Rₐ; the "substituted" refers to being substituted by one or more groups selected from the group consisting of D, halogen, amino, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
unless otherwise specified, the above alkyl, alkoxy, alkenyl, alkynyl, heterocycloalkyl, cycloalkyl, heteroaryl, and aryl may be further optionally substituted by one or more Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
m is 1, 2, 3, 4, 5 or 6;
n is 0, 1, 2, or 3.

2. The compound or the stereoisomer or optical isomer, pharmaceutically acceptable salt, prodrug or solvate thereof of claim 1, wherein the compound has a structure shown in formula II: 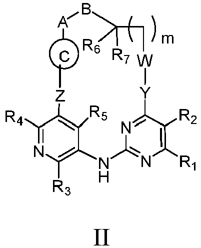 wherein, A, B, C, W, Y, Z, R₁, R₂, R₃, R₄, R₅, R₆, R₇, and m are as defined in claim 1.

3. The compound or the stereoisomer or optical isomer, pharmaceutically acceptable salt, prodrug or solvate thereof of claim 1, wherein ring C is selected from the group consisting of 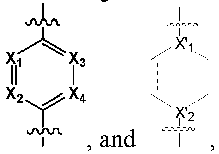
wherein, -̅-̅-̅-̅-̅ represents single bond or double bond;
X₁, X₂, X₃, X₄, X'₁ and X'₂ are each independently selected from the group consisting of N, and C-R_{d}; and 0, 1, 2 or 3 of X₁, X₂, X₃, and X₄ are N;
R_{d} is as defined in claim 1.

4. The compound or the stereoisomer or optical isomer, pharmaceutically acceptable salt, prodrug or solvate thereof of claim 1, wherein the compound has a structure shown in formula III: 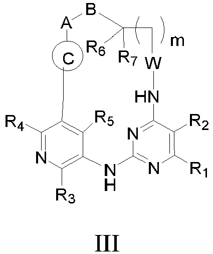 wherein, A, B, C, W, R₁, R₂, R₃, R₄, R₅, R₆, R₇ and m are as defined in claim 1.

5. The compound or the stereoisomer or optical isomer, pharmaceutically acceptable salt, prodrug or solvate thereof of any one of claims 1-4, wherein, ring C is selected from 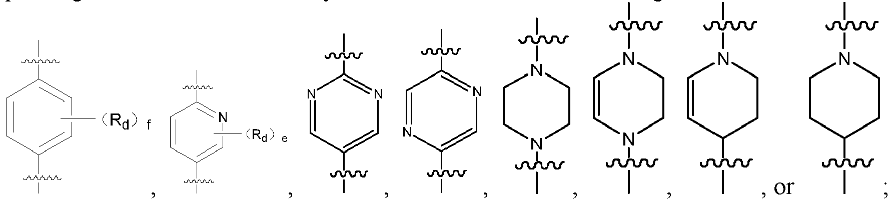
R_{d} is each independently selected from the group consisting of H, D, halogen, amino, nitro, hydroxyl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
e is 0, 1, 2 or 3;
f is 0, 1, 2, 3 or 4.

6. The compound or the stereoisomer or optical isomer, pharmaceutically acceptable salt, prodrug or solvate thereof of any one of claims 1-5, wherein the compound has a structure shown in formula IV-1 and IV-2: 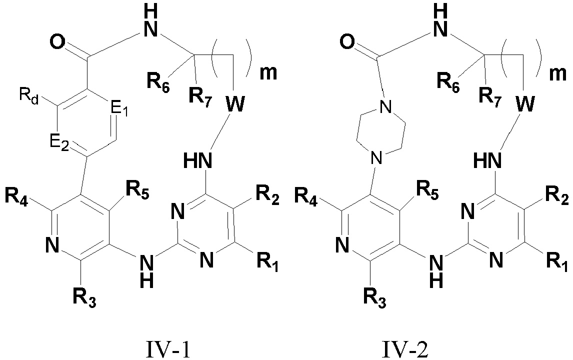 wherein,
E₁ and E₂ are each independently N or CR_{d};
R₁, R₂, R₃, R₄, R₅, R₆, R₇, R_{d}, and m are as defined in claim 1.

7. The compound or the stereoisomer or optical isomer, pharmaceutically acceptable salt, prodrug or solvate thereof of claim 1, wherein, W is independently selected from the group consisting of C(R_{c})₂, 6 membered heterocycloalkyl, C6 cycloalkyl, 6 membered aryl or heteroaryl, and Lᵢ-(CH₂)ₙ-L₂; wherein L₁ is independently selected from the group consisting of absent, 6 membered heterocycloalkyl, C6 cycloalkyl, and 6 membered aryl or heteroaryl, L₂ is independently selected from the group consisting of N-R_{b}, C(=O)O-, O, S, SO, and SO₂;
the above heterocycloalkyl, cycloalkyl, heteroaryl, and aryl may be further optionally substituted by one or more Rₐ, wherein each Rₐ is independently selected from the group consisting of halogen, amino, nitro, hydroxyl, sulfydryl, cyano, carboxyl, sulfonyl, sulfinyl, amido, sulfonamido, ester group, formyl, formamido, C1-C6 alkyl, C1-C6 alkoxy, C2-C6 alkenyl, C2-C6 alkynyl, 3-10 membered heterocycloalkyl, C3-C10 cycloalkyl, 5-12 membered heteroaryl, and C6-C12 aryl;
R_{b}, R_{c}, and n are as defined in claim 1.

8. The compound or the stereoisomer or optical isomer, pharmaceutically acceptable salt, prodrug or solvate thereof of claim 1, wherein the compound is selected from the group consisting of

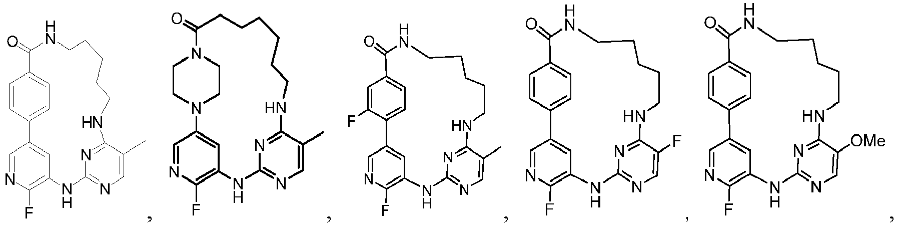

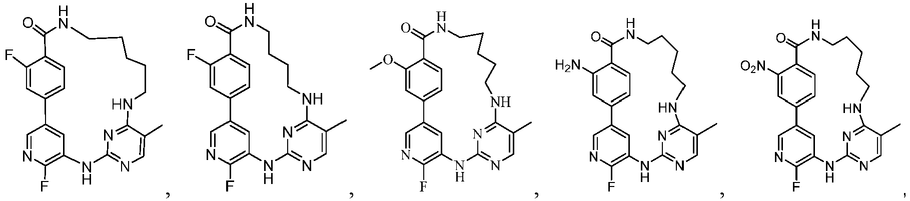

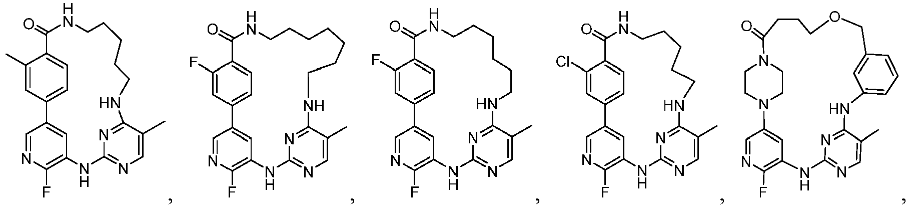

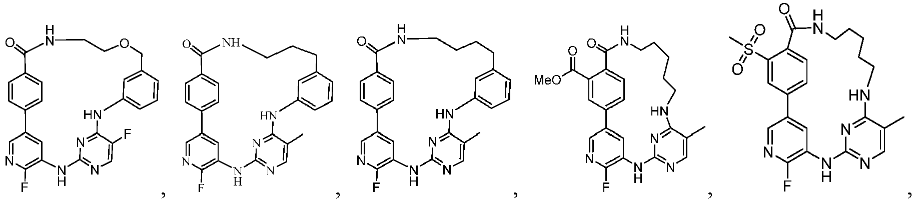

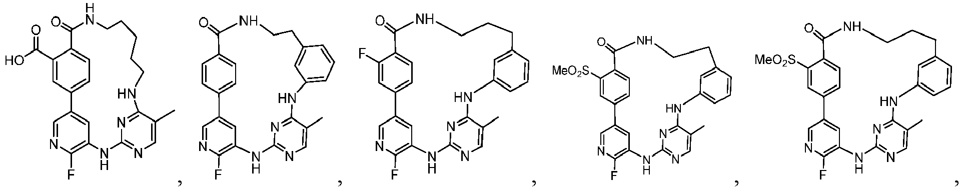

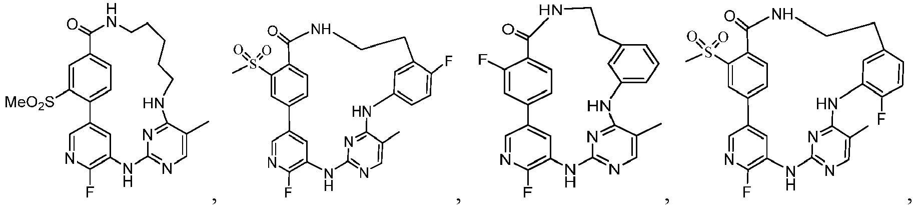

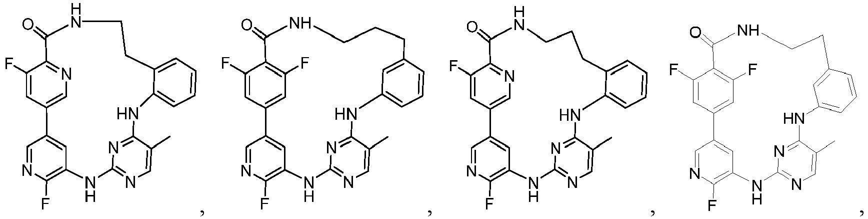

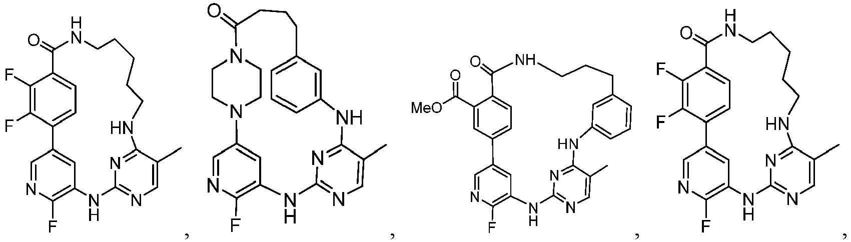

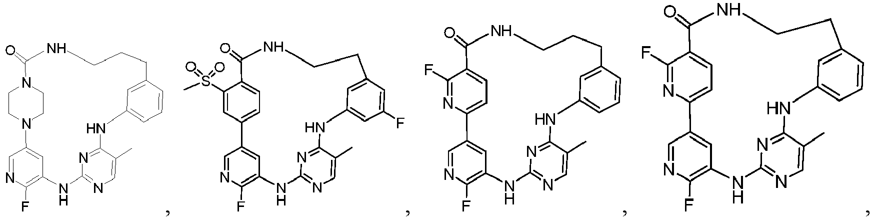

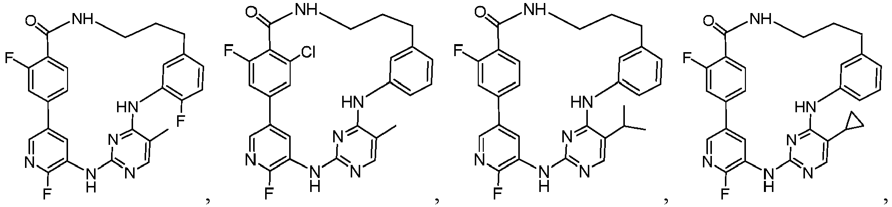

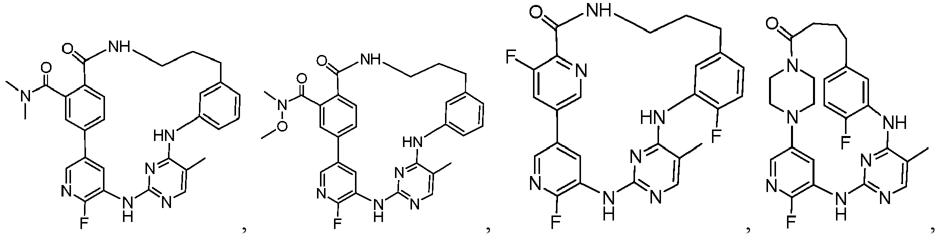

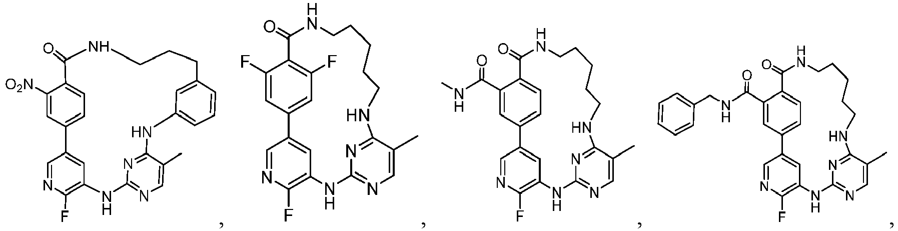

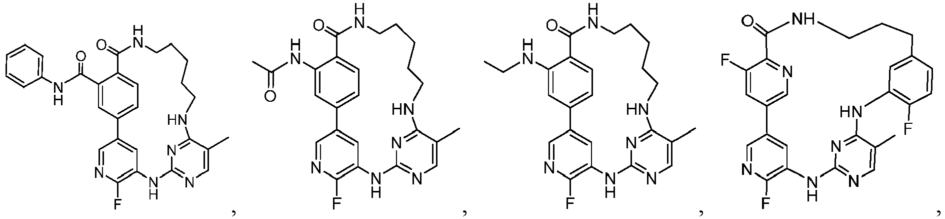

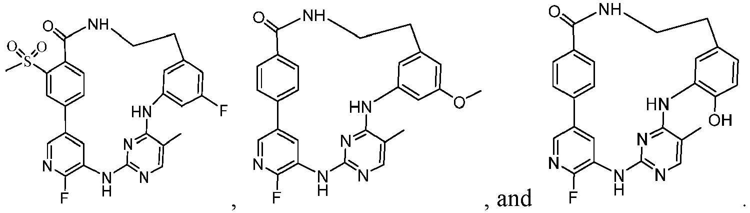

9. A pharmaceutical composition comprising the compound, or the stereoisomer or optical isomer, pharmaceutically acceptable salt, prodrug or solvate thereof of any one of claims 1-8; and a pharmaceutically acceptable carrier.

10. The pharmaceutical composition of claim 7, wherein the pharmaceutical composition is used for treating or preventing diseases associated with the activity or expression of JAK kinase.

11. Use of the compound or the stereoisomer or optical isomer, pharmaceutically acceptable salt, prodrug or solvate thereof of any one of claims 1-8 for preparing a medicament or a pharmaceutical composition for treating or preventing a disease related to the activity or expression of JAK kinase.

12. The use of claim 11, wherein the disease is selected from the group consisting of cancer, myeloproliferative disease, inflammation, immune disease, organ transplantation, viral disease, cardiovascular disease or metabolic disease, human or animal autoimmune disease, rheumatoid arthritis, skin disease, multiple sclerosis, rheumatoid arthritis, psoriatic arthritis, inflammatory bowel disease, myasthenia gravis, and psoriasis.
